# EUROPEAN PATENT APPLICATION

(11) **EP 2 623 985 A1**
(43) Date of publication of application: **07.08.2013**
(21) Application number: 13152802.8
(22) Date of filing: 19.02.2009
(51) Int. Cl.: G01N 33/574

(54) **Method for early determination of recurrence after therapy for prostate cancer**

(30) Priority: 21.02.2008 US 30462 P; 21.02.2008 US 66732 P; 22.02.2008 US 30718 P
(62) Divisional of application: 09713301.1
(71) Applicant: Iris International, Inc., Chatsworth, CA 91311 (US)
(72) Inventor: Sarno, Mark J., Encinitas, CA California 92024 (US); Klem, Robert E., Rancho Santa Fe, CA California 92067 (US); Saunders, Russ, Carlsbad, CA California 92004 (US); Jablonski, Edward, Escondido, CA California 92029 (US); Adams, Thomas, Rancho Santa Fe, CA California 92067 (US)
(74) Representative: Harrison Goddard Foote LLP

(57) **Abstract**

This invention describes compositions and methods for use in PSA assays having low functional sensitivity which are useful, for example, in the detection of early stage recurrence of prostate disease following treatment and in the determination of whether patients have early stage biochemical reoccurrence (ES-BCR) or stable disease. exponential fit

## Description

### FIELD OF THE INVENTION

This invention relates to compositions and methods useful in the detection of early stage recurrence of prostate disease following treatment.

### BACKGROUND AND INTRODUCTION TO THE INVENTION

Worldwide, there are approximately 670,000 new cases of prostate cancer per year. UK Prostate Cancer incidence statistics,
http://info.cancerresearchuk.org/cancerstats/types/prostate/incidence/ (last accessed January 23, 2009). In Europe in 2004, 237,800 new cases were diagnosed and 85,200 deaths occurred due to prostate cancer. Boyle, P et al. Annals of Oncology 16:481-488 (2005). In addition to clinical risk factors such as family history of cancer, smoking status, age, and race, initial detection of prostate cancer is generally based upon findings of increased circulating concentrations of a protein called Prostate-Specific Antigen (PSA), a neutral serine protease produced by normal, benign and malignant prostatic epithelial cells. PSA produced by prostatic cells is present in both free and complexed forms in seminal fluid, serum, plasma, and urine and can be measured in those fluids. Simultaneous measurement of the free and complexed forms is called "total PSA" measurement and may be referred to correctly as either "tPSA" or "PSA." The concentration of PSA in blood increases in various prostate diseases, particularly in prostate cancer, and this increased concentration is reflected in serum measurements of PSA. Valsanen et al., Prostate Cancer and Prostatic Disease 2:91-97 (1999). Thus, for the past two decades, assays such as conventional immunoassays for serum PSA have been used in the initial detection of prostate cancer. Yu et al., J. Urology 157:913-918 (1997).

Generally, if increased serum PSA concentrations are observed in a patient, a prostate biopsy is performed to confirm the presence of cancer and to characterize the cancer pathology. Once prostate cancer is confirmed, approximately two-thirds of patients are treated with radical prostatectomy (RP, the complete surgical removal of the prostate), or radiation, hormonal, or chemotherapies by a variety of methods. However, up to 40% of those treated patients may undergo disease recurrence. *See* Moul, J. Urology 163:1632-1642 (2000). Recurrence of prostate cancer is associated with a poor prognosis for survival. However, prognosis can be improved if the recurrence is detected at an early stage so that appropriate management methods including salvage treatments may be initiated. Unfortunately, existing methods for evaluating the likelihood of recurrence are insufficient for early detection. Clinicopathological observations taken prior to, or at the time of RP such as cancer stage, Gleason score, age at diagnosis, surgical margin involvement (presence of cancer at the surgical margin), local tissue invasion of the cancer, prostate capsule invasion of the cancer, seminal vesicle invasion of the cancer, bladder neck invasion of the cancer, lymph node invasion of the cancer, and total tumor volume are somewhat informative in assessing the likelihood of disease recurrence but are not always predictive and cannot be used to identify the exact time of a recurrence. Biopsy or imaging methods of various types can be used to confirm disease recurrence but these methods suffer from poor sensitivity. Generally, by the time a biopsy or imaging study detects new tumors, the recurrence is at a late stage when prognosis is especially poor. Thus, these methods are insufficient for early detection and aggressive treatment based thereon.

To address the insufficiencies of basing disease recurrence on clinicopathological findings and biopsy or imaging studies, disease recurrence is now primarily based upon findings of increasing serum PSA concentrations in the patient following treatment. For example, following a radical prostatectomy where no residual, PSA-secreting prostate tissue remains and sufficient time has passed for the physiological clearance of pre-operative levels of PSA, the serum concentration of PSA falls to a nadir. If the serum PSA concentrations should begin to rise after the nadir point, a disease recurrence may be indicated. This type of recurrence is referred to as a "biochemical recurrence" (BCR) in that the recurrence reflects only an increase in circulating levels of PSA rather than new findings of local or distant tumors. Biochemical recurrence of PSA has become the current standard of care in medical management of prostate cancer following treatment such as RP.

Various thresholds have been published to establish the point at which biochemical recurrence is thought to occur. Cookson MS, et al. J Urology 177:540-545 (2007). Typically, a value of 200 pg/mL (0.2 ng/ml) following the nadir of PSA is utilized to define the point of biochemical recurrence. *Id*. Conventional assays for PSA have detection limits in the range of 100 pg/ml with functional sensitivities possibly higher. The mean detection time for biochemical recurrence using a conventional PSA assay with a detection limit of 100 pg/mL is over 38.4 months. Vassilikos et al., Clinical Biochemistry 33(2): 115-123 (2000).

### BRIEF SUMMARY OF THE INVENTION

This invention is useful in the monitoring of patients treated for prostate disease, and the detection of prostate cancer, and cancer recurrence or stable disease following therapy, or following a decision not to administer post-prostatectomy therapy depending on clinical observations and the PSA values and PSA indicators of this invention. The present invention has advantages over conventional serum PSA assays for identification of biochemical recurrence of prostate cancer following treatment by providing novel assays with limits of detection and functional sensitivities for PSA superior to conventional assays. This invention is therefore useful in the monitoring of patients treated for prostate disease and the detection of cancer recurrence as opposed to stable disease (absence of recurrence) following primary therapy such as RP.

The methods described herein are also useful, for example, in detecting early stage recurrence of prostate cancer or to make early determinations that a patient is stable following radical prostatectomy for prostate cancer. The improved limit of detection and functional sensitivity of the present invention enables early detection of recurrence and, in appropriate cases, enables early initiation of salvage therapies for recurring cancer.

Therapy for prostate cancer may be radical prostatectomy, radiation therapy, chemotherapy, or anti-androgen treatment. Early detection of stable disease can avoid unnecessary adjuvant therapies in relatively young patients with poor margins and Gleason scores, who would otherwise be treated if stable disease were not detected. On the other hand, patients for whom early stage recurrence is detected using the methods of this invention, can undergo earlier treatment. Thus, the ability to detect low levels of PSA would allow one to reduce therapy of some patients who are currently being treated because they have a high probability of relapse, because one would now know they are not having of BR because their PSA level is low. Also this early detection of BR would lead to early therapy. Nilsson et. al., Acta Oncologica Vol. 43, No. 4, pp 316-381, 2004.

In one embodiment level concentrations of total PSA (tPSA or PSA) can be monitored in a patient following therapy, by obtaining one or more samples from the patient after the therapy and determining the amount of PSA in each sample using a PSA assay having a detection limit at least as low as 1 pg/mL and a functional sensitivity lower than 10 pg/mL. In another embodiment, a PSA assay having a detection limit and functional sensitivity of less than 1 pg/mL is used to determine recurrence of prostate cancer in a patient after therapy by determining whether a PSA value exceeds its corresponding PSA indicator cutoff. In a more preferred embodiment the PSA assay has a detection limit at least as low as 0.2 pg/mL and a functional sensitivity equal to or lower than 0.5 pg/mL.

The improved limit of detection and functional sensitivity of the PSA assays used in the methods of this invention permit detection of biochemical relapse or recurrence at an earlier stage. This detection of early stage biochemical recurrence should permit salvage therapies at an earlier stage, when there are fewer cancer cells and such cells may be more sensitive to treatment. Salvage treatments may include localized radiotherapy, and may be administered with or without concurrent androgen deprivation. For example, salvage radiotherapy has been shown to have a beneficial effect when used in treating men with PSA doubling times (the time in days or months or years when doubling of serum PSA concentration occurs) of less than 6 months, when the treatment was given < 2 years after biochemical recurrence determined using standard conventional assays. Trock et al., ASCO 2008 Urogenitary Cancers Symposium, Abstract No. 85. In addition, detection of early stage biochemical recurrence may eliminate the need to conduct further costly management in patients who have stable disease, or avoid the need for unnecessary adjuvant and salvage therapies in those patients.

In another embodiment of this invention assays for PSA having a functional sensitivity of at least less than 1 pg/mL are used to detect biochemical recurrence at an early stage following therapy for prostate cancer. Indicators based on PSA measurements are used in the detection of early stage biochemical recurrence. These indicators include the maximum observed PSA level during monitoring, the nadir PSA level, a multiplier of the nadir PSA level, ratio of maximum observed PSA level to nadir PSA level, or the number of doublings. PSA rate indicators such as velocity of PSA increase slope of Ln [PSA] vs. time, second consecutive increase (pg/mL/month), and doubling time can also be used. Any of these indicators can be used singly or in combination in determining whether a patient has early stage biochemical recurrence (ES-BCR), or stable disease.

In one aspect the PSA assays embodied in this invention are used to determine whether a patient has an early risk for prostate cancer recurrence, i.e., to detect early stage biochemical recurrence (ES-BCR), or whether the patient is more likely to display stable disease characteristics, i.e., to detect stable disease. For example, if the maximum observed [PSA] is equal to or exceeds a [PSA] indicator, it is determined that the patient has ES-BCR, and if the maximum observed [PSA] is less than a [PSA] indicator, it is determined that the patient has stable disease.

As another example, PSA assays can be used to measure the PSA concentration level in serial samples obtained from a patient following radical prostatectomy for prostate cancer. The measurements can be used to determine a PSA rate value. By determining whether the PSA rate of increase value is equal to or exceeds the PSA rate of increase indicator, it is possible to detect whether the patient has ES-BCR or stable disease. If the rate of increase in PSA is equal to or exceeds a rate indicator, it is determined that the patient has ES-BCR, and if the rate of increase in PSA falls below the threshold, it is determined that the patient has stable disease. When the PSA rate indicator is doubling time, the doubling time value is equal to or exceeds the doubling time indicator when the doubling time value is at least as low as the doubling time indicator, i.e. lower doubling times are associated with poorer prognosis than higher doubling times.

In another aspect, further analysis based on one or more PSA indicators permits classification of patients into additional sub-types, allowing clinicians to tailor treatments appropriate for that subtype and to use these therapies at an earlier time than current clinical practice. Early initiation of salvage treatment may improve patient outcomes.

The present invention will now be described more fully with reference to the accompanying figures and examples, which are intended to be read in conjunction with both this summary, the detailed description, and any preferred and/or particular embodiments specifically discussed or otherwise disclosed. This invention may, however, be embodied in many different forms and should not be construed as limited to the embodiments set forth herein; rather, these embodiments are provided by way of illustration only and so that this disclosure will be thorough, complete, and will fully convey the full scope of the invention to those skilled in the art.

### Description of the Figures

Figure 1 displays results from one embodiment of this invention and specifically shows the plot of the Nucleic Acid Detection Immunoassay, NADIA® [PSA] (PSA concentration) in pg/mL vs. days post radical prostatectomy for recurring patient number 11, with exponential fit. The NADIA® assay [PSA] was the [PSA] determined in the NADIA® assay study, described in the detailed description.

Figure 2 shows the plot of the NADIA® [PSA] in pg/mL vs. days post radical prostatectomy for recurring patient number 31.

Figure 3 shows the plot of the NADIA® [PSA] in pg/mL vs. days post radical prostatectomy for recurring patient number 38.

Figure 4 shows the plot of the NADIA® [PSA] in pg/mL vs. days post radical prostatectomy for stable patient number 86.

Figure 5 shows the plot of the NADIA® [PSA] in pg/mL vs. days post radical prostatectomy for stable patient number 120.

Figure 6 shows the plot of the NADIA® [PSA] in pg/mL vs. days post radical prostatectomy for stable patient number 126.

Figure 7 shows the plots in pg/mL vs. days post radical prostatectomy for all 43 recurring patients are shown in the Figure.

Figure 8 shows an overlay plot for 43 recurring patients, of [PSA] pg/ml vs time following prostatectomy with the PSA level range constrained to 1000 pg/ml, .

Figure 9 shows a plot of the first post-prostatectomy total [PSA] vs. the patient sub-population (recurrence of prostate cancer (1) or with stable disease (0)).

Figure 10 shows a plot of the nadir total [PSA] vs. the patient sub-population (recurrence of prostate cancer (1) or with stable disease (0)).

Figure 11 shows a plot of the maximum observed [PSA] level (pg/mL) vs. the patient sub-population (recurrence of prostate cancer (1) or with stable disease.(0)).

Figure 12 shows a plot of the maximum [PSA] level /nadir level [PSA] vs. the patient sub-population (recurrence of prostate cancer (1) or with stable disease (0)).

Figure 13 shows a plot of the second consecutive increase in [PSA] level (pg/mL/month) vs. the patient sub-population (recurrence of prostate cancer (1) or with stable disease (0)).

Figure 14 shows a plot of the doubling time data (days) vs. the patient sub-population (recurrence of prostate cancer (1) or with stable disease (0)).

Figures 15A-C show the overlay plots for recurring patients with doubling times of < 150 days, 150-400 days, or > 400 days, respectively.

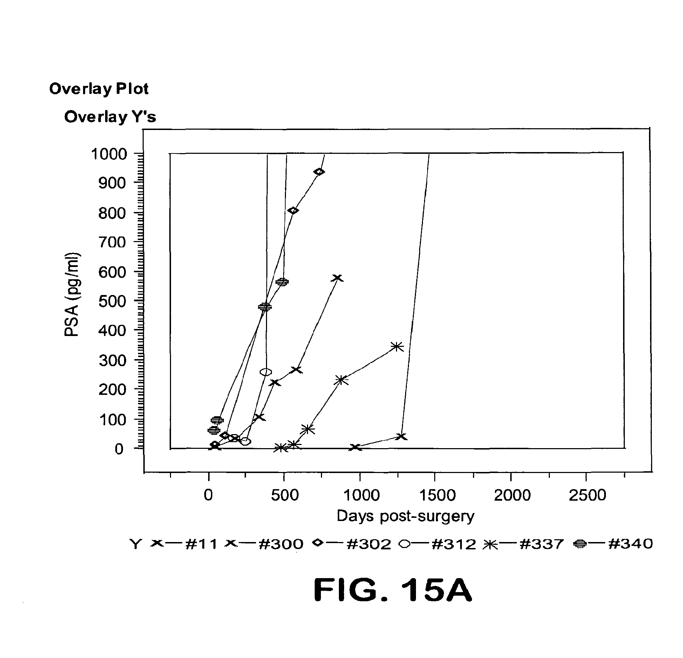

Figure 15A shows the overlay plots for recurring patients, of [PSA] pg/ml vs days post surgery with doubling times of < 150 with range constrained to 1000 pg/mL

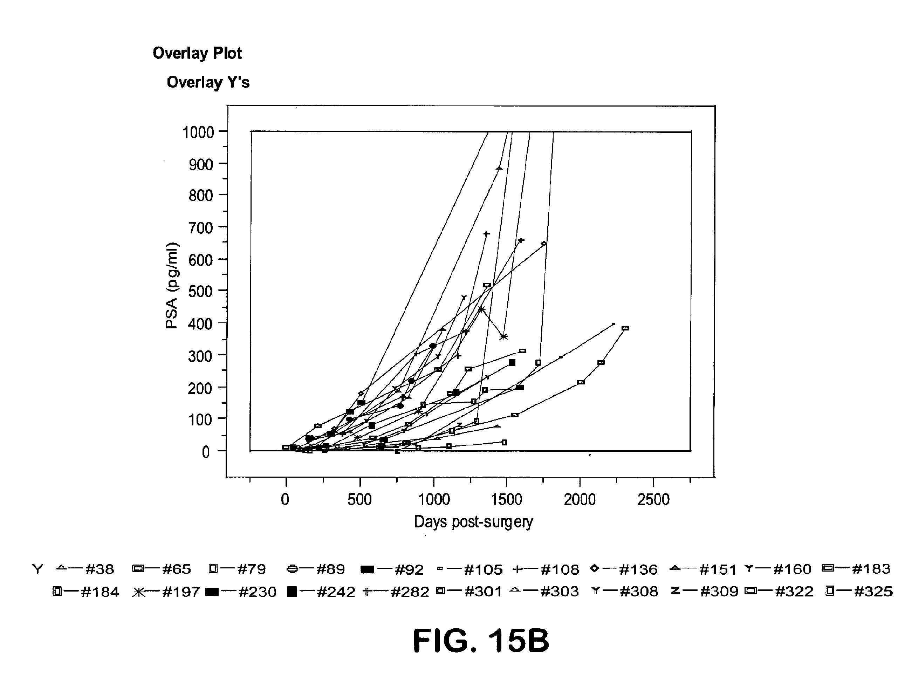

Figure 15B shows the overlay plots for recurring patients, of [PSA] pg/ml vs days post surgery with doubling times of 150-400 with range constrained to 1000 pg/mL

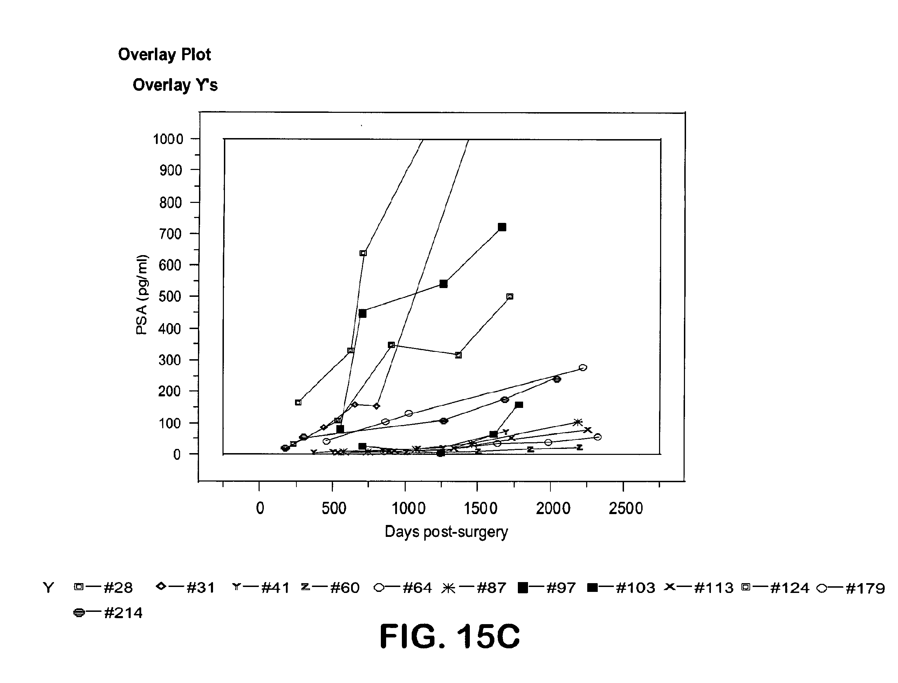

Figure 15C shows the overlay plots for recurring, of [PSA] pg/ml vs days post surgery patients with doubling times of >400 with range constrained to 1000 pg/M1

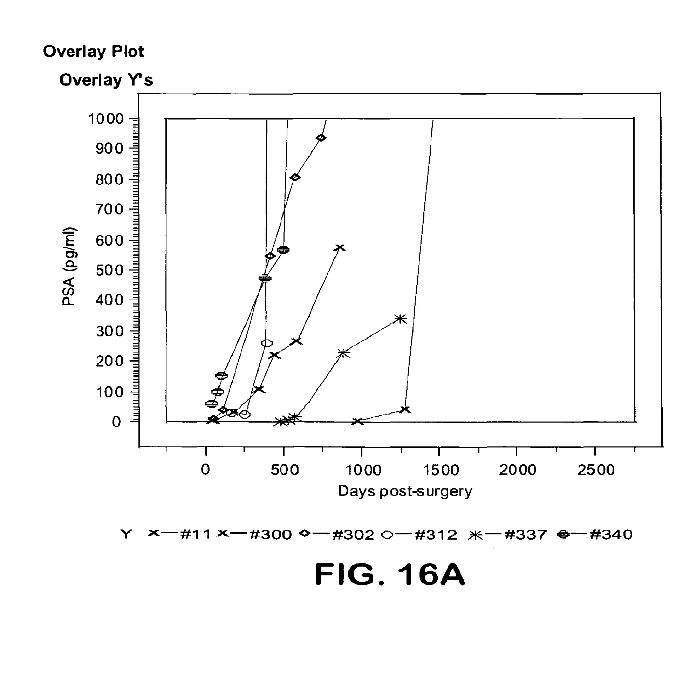

Figures 16A-D shows the overlay plots for subclasses of recurring patients by doubling time, with ranges constrained to 1000 pg/mL, respectively. The recurring patients with doubling times of > 400 days have been further subdivided whether the maximum observed PSA is above or below 200 pg/mL.

Figure 16A shows the overlay plots for recurring patients with doubling time < 150 days of [PSA] pg/ml vs days post surgery.

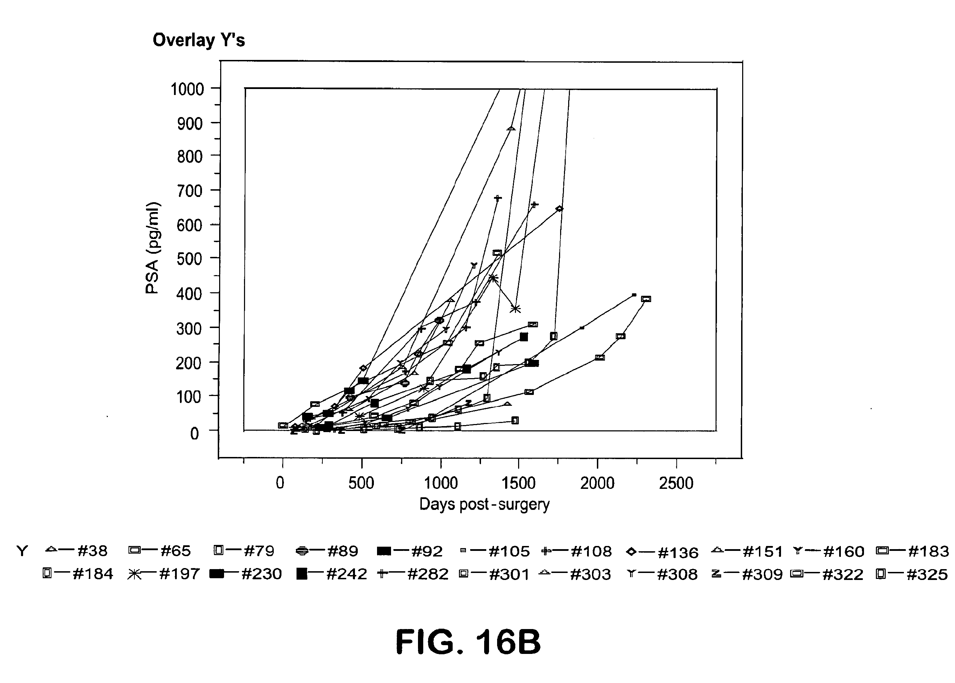

Figure 16B shows the overlay plots for recurring patients with doubling time < 150-400 days of [PSA] pg/ml vs days post surgery.

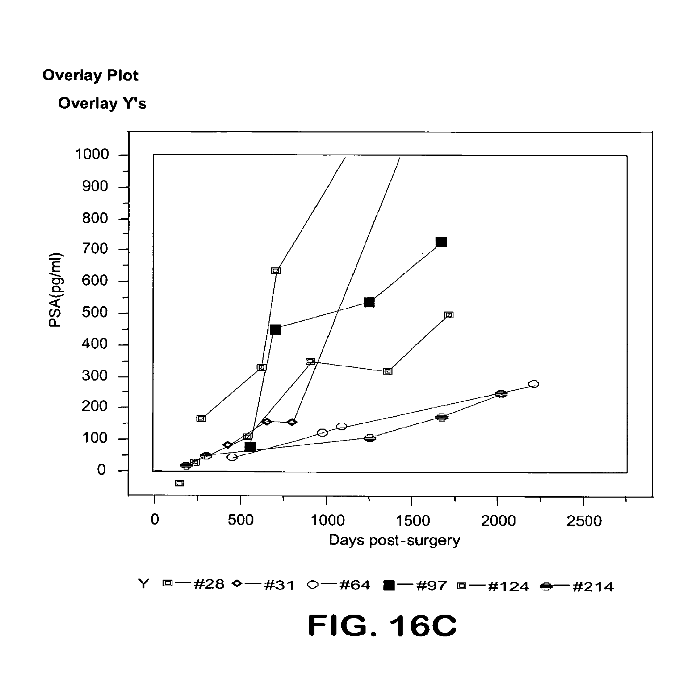

Figure 16C shows the overlay plots for recurring patients with doubling time> 400 days, maximum [PSA] > 200 pg/mL vs days post surgery.

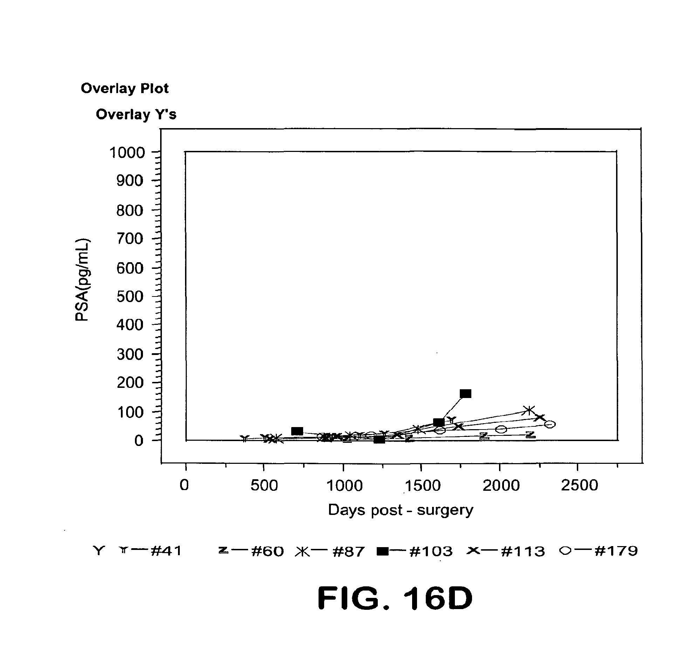

Figure 16D shows the overlay plots for recurring patients [PSA] pg/ml vs days post surgery.

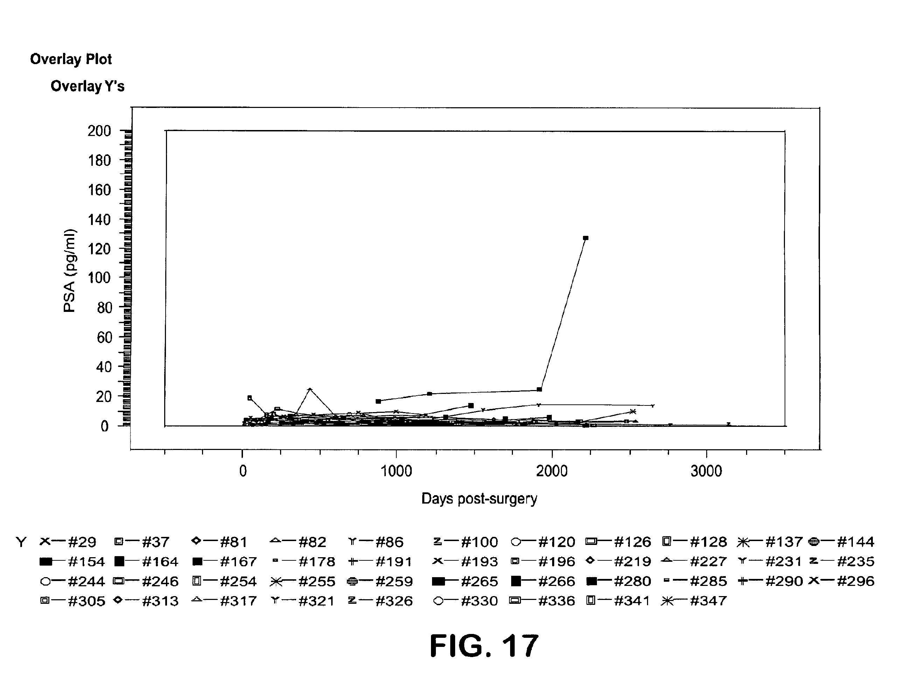

Figure 17 shows the overlay plots of [PSA] pg/ml vs days post surgery that, with few exceptions, the stable disease patients generally have PSA maximums which do not exceed 15 pg/mL.

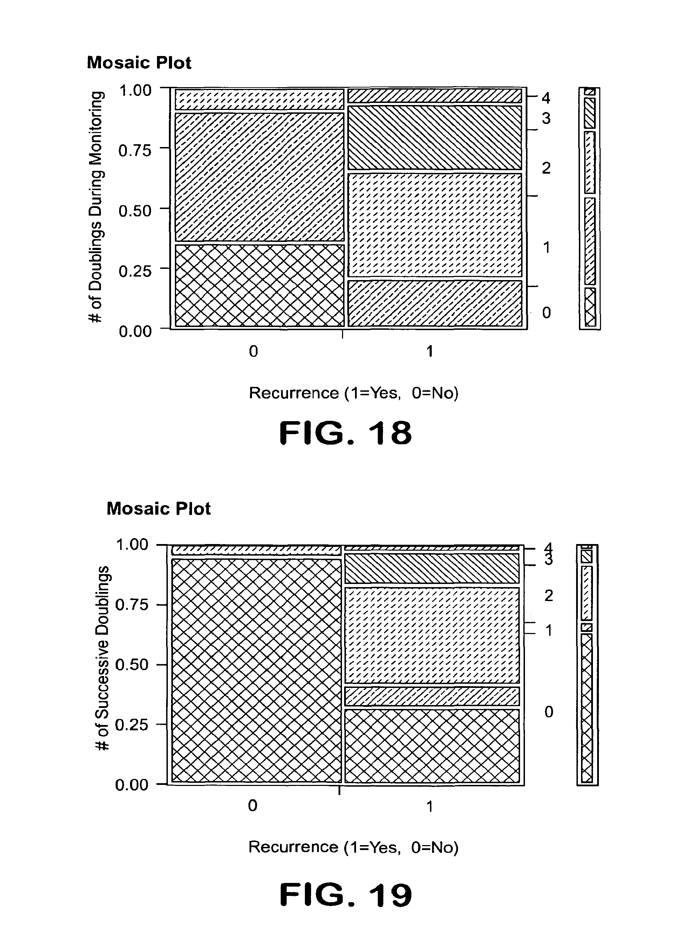

Figure 18 shows a mosaic plot of the data showing the number of doublings during monitoring vs. the patient sub-population (recurrence of prostate cancer (1) or with stable disease (0)).

Figure 19 shows a mosaic plot of the data showing the number of consecutive doublings vs. the patient subpopulation of recurrence of prostate cancer (1) or with stable disease (0).

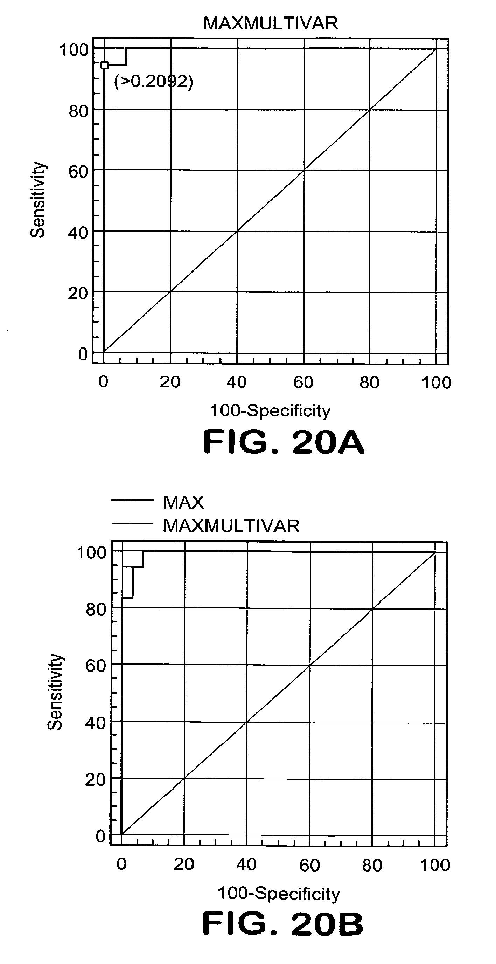

Figures 20A and 20B show the multivariate ROC curve in comparison to the univariate ROC curve for the NADIA® maximum observed [PSA] level. Figure 20A shows the multivariate ROC curve. Figure 20B shows the univariate ROC curve for the NADIA® maximum observed [PSA] level (black line) vs. the multivariate ROC curve (dotted line).

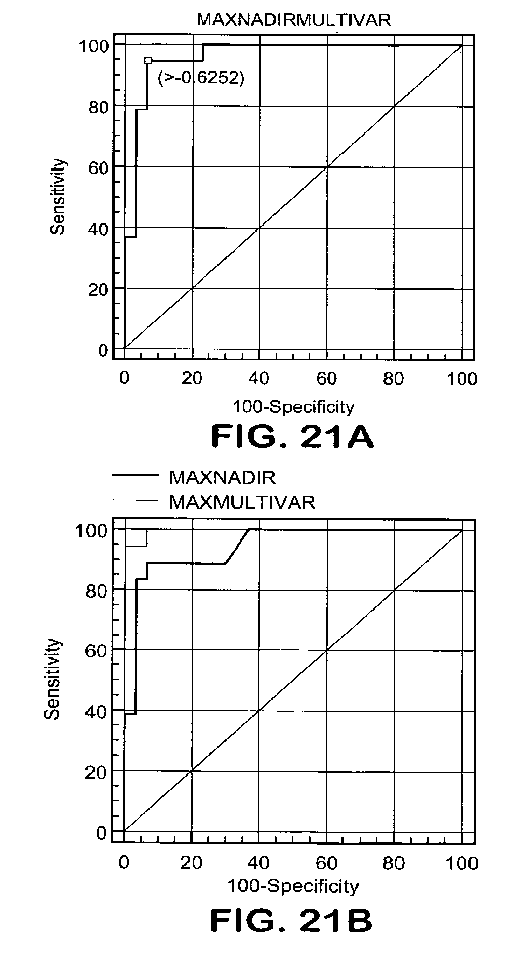

Figures 21A and 21B show the multivariate ROC curve in comparison to the univariate ROC curve for the NADIA® maximum total [PSA]/nadir [PSA] levels. Figure 21A shows the multivariate ROC curve. Figure 21B shows the univariate ROC curve for the NADIA® maximum total [PSA]/nadir [PSA] levels (black line) vs. the multivariate ROC curve (dotted line).

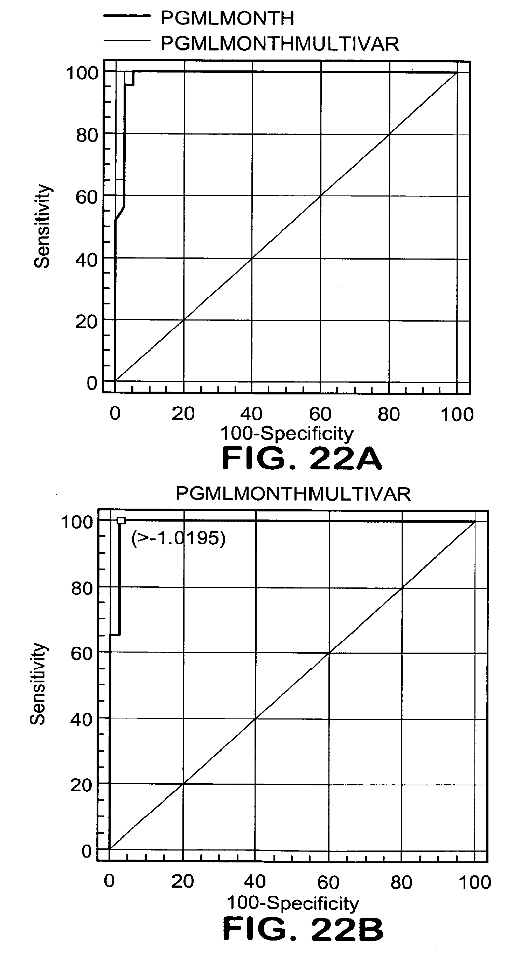

Figures 22A and 22B show the multivariate ROC curve in comparison to the univariate ROC curve for the second rise in [PSA] (pg/mL/month). Figure 22B shows the multivariate ROC curve. Figure 22A shows the univariate ROC curve for the NADIA® second rise in [PSA] (pg/mL/month) (black line) vs. the multivariate ROC curve (dotted line). Table 22 shows the results of the logistic regression and ROC computations.

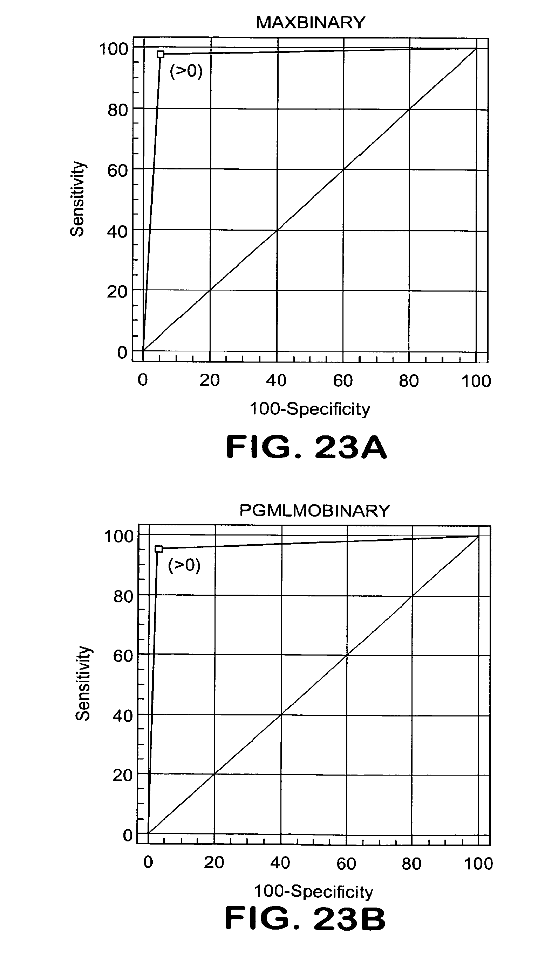

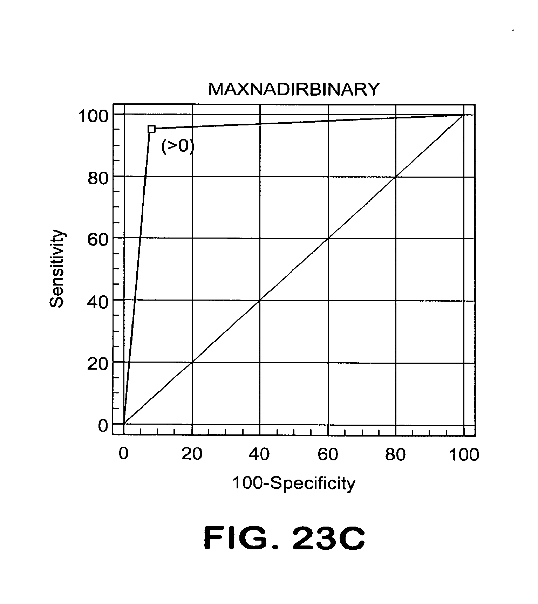

Figures 23A-C show the univariate analysis for maximum total [PSA], second rise (pg/mL/month) indicators, and maximum total [PSA]/nadir total [PSA].

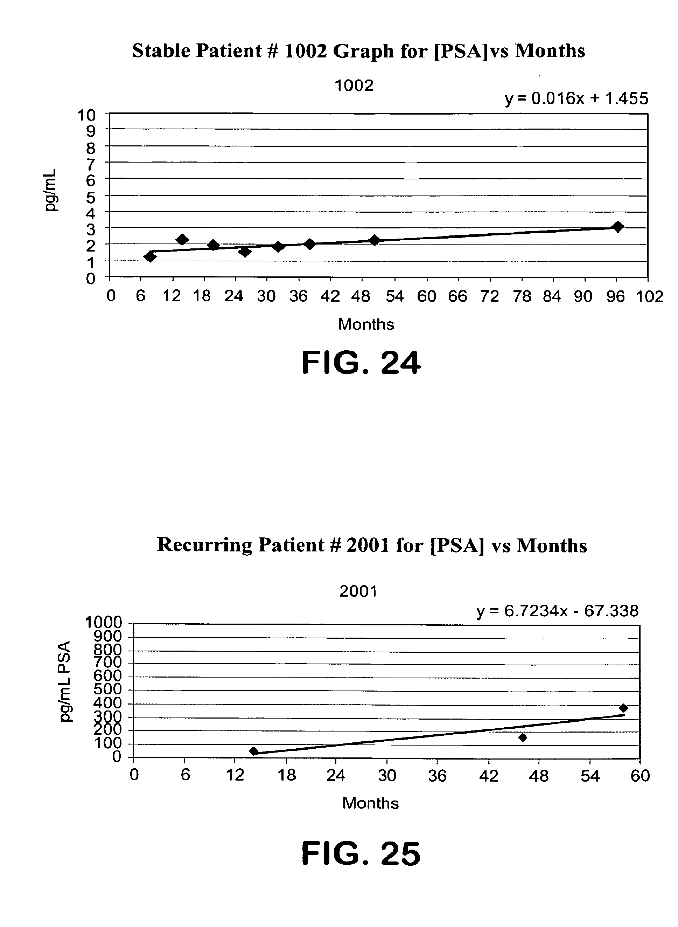

Figure 24 shows a linear curve fit for a stable patient for level of [PSA] (pg/mL) vs. time (months) over a time period of approximately eight years.

Figure 25 shows a linear curve fit for a recurring patient for level of [PSA] (pg/mL) vs. time (months) over a time period of approximately five years.

### DETAILED DESCRIPTION OF THE INVENTION

According to this invention, assays for total serum PSA (total serum PSA is the simultaneous measurement of both free and complexed forms of PSA in serum) having a detection limit at least as low as 1 pg/mL and a functional sensitivity at less than 10 pg/mL are used to monitor patients following therapy for prostate cancer, and can be used to detect early stage biochemical recurrence following therapy as opposed to stable disease post-surgery.

However, there is a limitation even to the use of biochemical recurrence as an indicator of prostate cancer recurrence when conventional assays for PSA are used. The lowest values of serum PSA following radical prostatectomy are often below the limits of detection when conventional assays are used to measure PSA. *See* Junker et al., Anticancer Research 19:2625-2658 (1999). Thus, values of serum PSA following RP may be reported as zero nanograms/milliliter (ng/ml) with conventional assays when PSA is not actually absent in the circulation. *See* Stamey, Clin. Chem. 42(6): 849-852. Even if the PSA value is above the detection limit of a conventional assay, the concentration may nevertheless be below the assay's "functional sensitivity," the ability to quantify concentrations of serum PSA at low levels with accuracy and precision. This means that the true nadir concentration of serum PSA either cannot be detected or cannot be reported with accuracy and precision by conventional assays. This is unfortunate since the nadir concentration itself may be a predictor of recurrence with lower nadir concentrations associated with lower likelihood of recurrence. Furthermore, if the serum PSA level should begin to rise, it may not be detectable by conventional assays until a time at which recurrence is at a stage when prognosis may again be poor.

Aggressive cancers may recur far more rapidly but conventional assays would not be able to detect these recurrences due to their limits of detection and insufficient functional sensitivity. Even non-aggressive cancers may begin to show a rise in serum PSA that is not detectable by conventional assays. Thus, conventional assays for serum PSA are not able to aid physicians in the early detection of prostate cancer recurrence.

Most current FDA approved conventional PSA assays measure down to approximately 100 pg/mL, and that limit of detection is reflected in the definition of biochemical recurrence recently recommended by the American Urological Association Prostate Cancer Guideline Panel ([PSA] of greater than 0.2 ng/mL (200 pg/mL), with a second confirmatory level of PSA greater than 0.2 ng/mL). See Cookson, et al., J. Urology 177:540-545 (2007). Due to the limitations in functional sensitivity, conventional PSA assays indicate the absence of PSA in samples having [PSA] below the functional sensitivity of the assays. E.g., Stamey (1996); Vassilikos et al., Clinical Biochemistry 33(2): 115-123 (2000).

For detection of early stage recurrence following therapy, it is of clinical importance to know whether PSA in post-therapy samples is within the functional sensitivity of an assay. Otherwise, clinicians and patients do not know whether a negative result reflects the "absence" of PSA or the limits of detection of the assay despite the presence of PSA-producing cells.

In the methods of this invention, assays having a low functional sensitivity limit as described herein have been used to measure PSA levels down to the 0.2-0.5 pg/mL range in serum samples from women. The 0.5 pg/mL functional sensitivity of the assay permitted determination that the levels of PSA in the sera of women are in the range of 0.5 to 3 pg/mL rather than zero, as was commonly assumed. It is expected that following radical prostatectomy, men have a PSA level at least equal to the levels found in women. Thus, the assays with functional sensitivity down to 0.5 pg/mL are capable of measuring the lowest levels of PSA that one would expect to find in men post radical prostatectomy.

Measuring PSA levels using PSA assays with a functional sensitivity of less than 0.5 pg/mL permitted precise measurement of the low PSA levels in post-therapy prostate cancer patients. Measurement of [PSA] using the Nucleic Acid Detection Immunoassay (NADIA® test) showed that following radical prostatectomy, many patients have stable low PSA levels, which indicates that those patients have very slow growing cancers or are cured. For patients displaying increased serum PSA levels with time, PSA levels were accurate. Patients' PSA serum levels were accurate enough to determine slopes for the increase in PSA, and to generate reproducible data for samples containing PSA levels previously below the functional sensitivity of current assays. Measuring the level of PSA refers to measuring the level of total PSA, or tPSA.

Use of the more sensitive PSA assays established that PSA levels increase exponentially following the post-RP nadir. The NADIA® PSA assay results indicated that cancer cells were present and growing exponentially long before the [PSA] level reached 200 pg/mL. The results from a retrospective analysis of a dataset shows that prostate cancer cells are present and growing for a considerable length of time before the serum level reaches the current biochemical recurrence point of 200 pg/mL.

In one aspect of the invention a PSA assay having a functional sensitivity of at least as low as 0.5 pg/mL and/or a detection limit of 0.2 pg/mL is used to determine recurrence of prostate cancer at an early stage. It also decreases the time needed to detect early stage recurrence, up to, for example, 30 months earlier than with conventional assays. Precise measurements of PSA in the 0.5 to 100 pg/mL range using these PSA assays also permits recognition of early stage biochemical recurrence and initiation of treatment much earlier than that based on current clinical practice.

Earlier detection of the need for salvage treatment for early stage recurrent prostate cancer decreases the time required to begin follow up treatment of patients, which generally currently takes place only after PSA levels exceed 200 pg/mL. As described herein, using PSA assays having a functional sensitivity of 0.5 pg/ml to monitor patients could lead to evaluations for further therapy at least as much as 30 months sooner than using current measures of biochemical recurrence. This will assist in providing earlier treatment when the cells are potentially more localized and/or susceptible to therapy.

In one aspect, the methods of this invention permit earlier and more accurate identification of men at risk for disease progression and patients with early treatment failure. The methods of this invention can also be used to earlier determine that the patient is not having a recurrence. The availability of more sensitive PSA assays therefore reduces system costs and patient anxiety by permitting earlier classification of patients as stable or having early stage biochemical recurrence.

In some aspects, the highly sensitive, early detection methods of this invention can be used in evaluating treatment options following radical prostatectomy. In some embodiments, this invention can be used to detect whether patients have stable disease, whether, and how often patients should be monitored for recurrence, and whether and when salvage treatments such as anti-androgen treatment, radiotherapy or chemotherapy should be administered.

Post prostatectomy treatments have been determined largely based on clinical observations such as Gleason score, age at diagnosis, surgical margins, T-stage, tissue invasion, capsular invasion, seminal vesicle invasion, bladder neck invasion, lymph node invasion, and tumor volume. Clinical parameters having predictive value for recurrence include high Gleason score, high PSA using current assays (above 200 ng/ml measured with current assays), pT3 disease, positive surgical margins and seminal vesicle invasion. See Nilsson at p. 346.

A high percentage of patients with prostate cancer are not cured by RP, and 27-53% will display elevated [PSA] within 10 years. Nilsson et al., "A systematic overview of radiation therapy effects in prostate cancer," Acta Oncologica, 43(4):316-381 (2004). However, between 30% and 70% of the patients currently treated with adjuvant therapy will not suffer from recurrence. Thus, administering adjuvant therapy to post-prostatectomy patients on the basis of clinical observations such as age, Gleason score and surgical margins alone may expose a significant percentage of patients who have stable disease to unnecessary, costly treatments and potential complications.

As an example, adjuvant treatments may be administered to patients displaying poor clinical signs. These patients include relatively young patients with poor margins and Gleason scores. For instance, patients in their fifties having poor margins and Gleason scores of >7, will usually undergo therapy such as external radiotherapy (RT). Post-prostatectomy treatment with external beam radiotheraphy in patients with stage pT3 disease prolongs biochemical disease-free survival, and the likelihood of achieving stable disease in patients who are not cured by RP is higher when treatment is given earlier, rather than delayed salvage therapy. See Nilsson et al., at 316.

However, use of the highly sensitive assays and [PSA] values and indicators of this invention can be used alone or in combination with clinical observations to provide early detection of stable disease, and can avoid unnecessary adjuvant therapies currently being administered. For example, early detection of stable disease in relatively young patients who would otherwise be treated, can avoid the need for unnecessary treatments, and attendant risk of side effects. Side effects of post-prostatectomy therapy can include incontinence, urinary frequency, nocturia, cystitis, diarrhea, rectal bleeding, decreased libido and/or impotence. Accordingly, in some aspects, early detection of stable disease using the detection methods of this invention can avoid unnecessary adjuvant therapies in patients who routinely currently receive adjuvant therapies based on clinical observations. On the other hand, delaying salvage treatment until the [PSA] obtained using conventional methods reaches 200pg.mL diminishes the likelihood of achieving stable disease. See Nilsson at 345.

Thus, in some aspects, the PSA values and PSA indicators of this invention are used in combination with clinical observations to determine whether adjuvant and/or salvage therapy should be administered. For example, if adjuvant and/or salvage therapy would normally be administered to a patient based on clinical observations, but one of more PSA values does not exceed the PSA indicator, and stable disease is detected, then unnecessary treatment could be avoided. PSA values and indicators that can be used in these methods are described throughout. As an example, when the [PSA] is lower than 15 pg/ml, and the slope of Ln [PSA] vs. time is lower than the slope of Ln [PSA] vs. time indicator, then even if a relatively young patient has poor margins and a Gleason score of >7, adjuvant treatment can be avoided, and the patient monitored until one or more [PSA] values exceeds the [PSA] indicator.

In other aspects, when the methods of this invention are used in combination with clinical observations to detect early stage recurrence, patients with ES-BCR can undergo earlier treatment, leading to increased prognosis. Radiation and chemotherapy can be performed according to methods and protocols known to those of skill in the art. Anti-androgen treatment can be performed using drug and biologic drug compositions, combinations, dosage forms and dosages known to those of ordinary skill in the art for adjuvant or salvage therapy in the treatment of post-prostatectomy patients.

An example of a PSA assay having a functional sensitivity of about 0.5 pg/mL and a detection limit of 0.2 pg/mL according to this invention is a sandwich format immunoassay using polymerase chain reaction (PCR) for signal generation. An example of such an assay useful in detecting PSA in serum or plasma samples in the methods of this invention is described below. Immuno PCR formats for assays for proteins are described in U.S. Patent No. 5,665,539, hereby incorporated by reference in its entirety. Any PSA assay having a functional sensitivity as least as low as specified may be used in the methods of this invention. Methods for detecting proteins and for signal generation in protein assays are known to those in the art. For example, the methods of this invention may use other assay formats, including sandwich immunoassay formats, and any method of signal generation capable of providing the required functional sensitivity for use in the methods of this invention. For example, the methods of signal generation may include use of deoxyribonucleic acid (DNA) arrays, bioluminescence, radioactivity, chemifluorescence, nanoparticles, or oligo-nanoparticles, either singly or in combination.

In addition, as discussed in more detail below, PSA values such as doubling time and/or maximum observed PSA concentration can be used to further classify early stage recurring patients into multiple groups. These classifications could potentially be used to recommend different therapies for patients in the different subgroups. Thus, use of the methods of this invention will provide clinicians and patients with an accurate indication of treatment failure or early stage biochemical recurrence, and will permit more timely and appropriate selection of therapies to control the disease. In addition, earlier treatment therapy as a result of early detection may improve patient outcomes and avoid the need for more costly management of patients having stable disease.

In one embodiment, this invention includes a method of detecting whether a patient has early stage biochemical recurrence (ES-BCR), comprising
a) obtaining a sample from a patient after therapy for prostate cancer;
b) measuring the PSA level in the sample using a PSA assay having a functional sensitivity at least as low as 20 pg/mL,
c) using the PSA level from one or more samples to determine a PSA value, wherein ES-BCR is detected if the PSA value exceeds a PSA indicator in one or more samples.

The assay for PSA can be used to determine the PSA level in samples taken from a patient following a treatment for prostate cancer. PSA level may include the amount or concentration of PSA in the sample. The sample may be a plasma or serum sample. Measurements of PSA levels may be used to monitor and assess whether therapy for prostate cancer has effectively treated the disorder. Preferably, the PSA assay has a functional sensitivity at least as low as 0.5 pg/mL and a detection limit as low as 0.2 pg/mL.

The "PSA value" is a parameter that is a function of the observed PSA level. PSA value may include, for example, the observed PSA level measured after the nadir PSA level, the ratio of the observed PSA level or maximum observed PSA level to the nadir PSA level, the slope of Ln [PSA] vs. time, the velocity of increase in PSA level, the doubling time for PSA level, or the second consecutive increase in PSA level. The observed PSA level may be a concentration or amount.

A "PSA indicator" is a predetermined cutoff, threshold or number, which discriminates with statistical significance between subpopulations of patients having stable disease and patients having, or who will have, biochemical recurrence and/or disease recurrence.

"Early stage biochemical recurrence" is detected when one or more selected PSA values obtained using a PSA assay with a functional sensitivity at least as low as 1 pg/mL exceed the corresponding PSA indicators. The values and corresponding indicators can be used singly or in combination in determining whether a patient has ES-BCR or stable disease.

Disease recurrence may be determined biochemically, or based on clinical observations such as imaging or biopsy, although those methods suffer from poor sensitivity for recurrence. One or more of the PSA values and PSA indicators obtained using the methods of this invention can also be used in combination with clinical observations to facilitate or determine treatment options for patients. For example, detection of ES-BCR using the methods of this invention may result in further therapy, including radiation therapy, chemotherapy or anti-androgen therapy. In some instances, further therapy may be warranted if there is an early, rapid, increase in a [PSA] value, and/or if an early measured PSA rate value exceeds a PSA rate indicator. As another example, an early, less rapid [PSA] rate increase may or may not result in further therapy, depending on other patient parameters including clinical observations. Clinical observations may include Gleason score, age at diagnosis, surgical margins, T-stage, tissue invasion, capsular invasion, seminal vesicle invasion, bladder neck invasion, lymph node invasion, biopsy, or tumor volume. In some embodiments, the parameters supporting further therapy include age less than an age cutoff, a Gleason score exceeding a Gleason score cutoff, high PSA using the methods of this invention, positive surgical margins and seminal vesicle invasion. The age cuttoff may be, for example, 50, 51, 52, 53, 54, 55, 56, 57, 58, 59 or 60, 65, 70, 75 or 80. The Gleason score cutoff may be, for example, 4, 5, 6, 7, 8, 9, 10.

As another example, a slow increase in a [PSA] rate value may not result in further therapy, if clinical observations indicative of lack of recurrence such as low Gleason score, or advanced age (such as over 70 or 80), are also present. In addition, if the methods of this invention detect stable disease, no further therapy will be administered. In any instance where further therapy is not administered, it may be desirable to further monitor one or more PSA values using the methods of this invention, either alone or in combination with clinical observations, to determine if further therapy should be administered at a later time.

A PSA indicator may be a predetermined cutoff or threshold for the maximum observed PSA level, a multiplier of the nadir PSA level, the maximum observed PSA level, the nadir PSA level, the slope of Ln [PSA] vs. time, the velocity of increase in PSA, or the doubling time for PSA. Doubling time is (Ln (2)/K), where K is the slope of the exponential fit of a plot of PSA level versus time. In the case of doubling time, the PSA value "exceeds" the PSA indicator when the doubling time value is less than or equal to the PSA indicator. The PSA indicators are determined using standard statistical methods such as those described herein. As an example, the PSA level indicator may be a [PSA] indicator of at least about 10, 11, 12, 13, 14, 15, 16, 17, 18, 19, 20, 21, 22, 23, 23, 24, 25, 26, 27, 28, 29, 30, 35, 40, 45, 50, 55. 60 pg/mL. More preferably, the PSA level indicator may be a [PSA] indicator of at least about 15 pg/mL, 20 pg/mL or 25 pg/mL. A PSA level indicator range may also be specified. A [PSA] indicator range may be, for example 15-25 pg/mL, 15-22 pg/mL or 20-25 pg/mL. The PSA level indicator may be used alone or in combination with other PSA indicators or clinical indicators to determine patients having stable disease or ES-BCR.

By "PSA nadir" is meant the lowest measured amount of PSA in a sample from the patient following therapy such as radical prostatectomy. The PSA nadir results from clearance of PSA produced by proliferating prostate tissue removed or killed during treatment. PSA has a half life of 2.2 days to 3.5 days, and may take from 3 to 4 weeks or up to 6-8 weeks to clear from the bloodstream. Ellis et al., Adult Urology, 50 (4), 573-579, (1997). Following treatment such as radical prostatectomy, the serum PSA level decreases to a nadir following treatment which removes or kills the proliferative prostatic cells. In patients with stable disease, the PSA levels may remain flat after reaching a low point. The sample may be one of a serial set of blood serum samples for which PSA level is measured. A serial set of blood serum samples is two or more samples taken at different time points from the same patient following therapy such as radical prostatectomy or salvage treatment.

Therapy refers to one or more treatments in the clinical management of prostate disease. A treatment for a prostate disease is preferably a treatment for prostate cancer. A treatment for prostate cancer is preferably a radical prostatectomy. Treatment for prostate cancer may also include radiation therapy, including salvage radiation therapy as well as hormonal or chemotherapies.

An assay for total PSA preferably has a detection limit at least as low as 10 pg/mL and a functional sensitivity at least as low as 20 pg/mL. A PSA assay may preferably have a functional sensitivity of at least as low as about 15, 14, 13, 12, 11, 10, 9, 8, 7, 6, 5, 4, 3, 2, 1, 0.9, 0.8, 0.7, 0.6, or 0.5 pg/mL. A PSA assay may preferably have a detection limit as low as 0.2 pg/mL and/or a functional sensitivity of about 0.5 pg/mL. The detection limit is alternatively referred to herein as functional detection limit or limit of detection. The limit of detection (LOD) is the lowest amount of analyte in a sample that can be detected with type I and II error rates set to 5%

In some embodiments the limit of detection can be at least as low as 15, 14, 13, 12, 11, 10, 9, 8, 7, 6, 5, 4, 3, 2, 1, 0.9, 0.8, 0.7, 0.6, 0.5 or 0.2 pg/mL. The PSA assay may also have a detection limit as low as 0.5 pg/mL, with a functional sensitivity as low as 1, 2, 3, 4, or 5 pg/mL. In some embodiments, the PSA assay has a functional detection limit of 0.2 pg/mL and a functional sensitivity of 0.5 pg/mL, and further comprises contacting the sample with a conjugate comprising a non-nucleic acid PSA binding entity and a nucleic acid marker that can be used to generate a PCR signal.

The most common definition of biochemical recurrence recently is a [PSA] of greater than 0.2 ng/mL (200 pg/mL), although levels ranging from 100 to 2000 pg/mL have been used. Doherty et al., J. Cancer 83(11): 1432-1436 (2000). With a PSA assay having a functional sensitivity of at least as low as 1.0 pg/mL, it is possible to determine whether or not early stage biochemical recurrence has occurred. The detection of early stage biochemical recurrence takes place earlier than detection of conventionally defined biochemical recurrence using conventional PSA assays.

In one aspect of the invention, ES-BCR based on PSA level may be detected by comparison of the maximum observed PSA level to a PSA level indicator. A PSA level of at least any level between 10 to 60 pg/mL, preferably 10, 11, 12, 13, 14, 15, 16, 17, 18, 19, 20, 21, 22, 23, 24, 25, 26, 27, 28, 29, 30, 35, 40, 45, 50, 55, or 60 pg/mL, and more preferably 10, 15, 20, 25, or 30 pg/mL can be used as a PSA indicator to determine ES-BCR. For example, if the maximum observed PSA level indicator is 15 pg/mL, then comparing a maximum observed PSA level greater than 15 pg/mL to the PSA indicator detects ES-BCR.

In another aspect, the maximum observed PSA to nadir PSA ratio may be used as the PSA indicator for determining whether ES-BCR has occurred. For example, the maximum observed PSA/nadir PSA may be any number between 3 to 11, preferably 3, 4, 5, 6, 7, 8, 9, 10, or 11, and more preferably 6. The multiplier of the nadir PSA level may be 2X, 4X, or 8X, preferably 4X.

In another embodiment of this invention, assays for PSA can be used to determine PSA in serial samples taken from a patient following therapy. PSA measurements from the serial samples taken over time can be used to calculate PSA rate values including velocity of the change in PSA, the slope of Ln [PSA] vs. time, or the doubling time for the increase in PSA. Comparison or one or more of these PSA rate values to its corresponding rate indicator permits determination of whether ES-BCR has occurred.

In this embodiment, the invention may comprise, for example, methods for determining whether a patient has early stage biochemical relapse (ES-BCR), comprising:
a) obtaining serial samples from the patient;
b) determining the PSA level in each sample using a PSA assay having a functional sensitivity at least as low as 1 pg/mL;
c) determining that the PSA rate value exceeds a PSA rate indicator, thereby detecting ES-BCR; or
determining that the PSA rate value does not exceed the rate indicator, thereby detecting that the disease is stable.

The first sample for use in determining a PSA value may be taken at any time after therapy, and at or following the clearance of pre-therapy PSA levels and the PSA nadir. Generally, the first sample will be taken any time between 2 weeks to 8 weeks following treatment. Samples may be taken at any set of intervals used in the clinical monitoring of prostate disease. Preferably the first sample will be taken 30 or 45 days after treatment, with subsequent samples preferably taken at 3 month intervals. This time course may be modified if the PSA value of a sample indicates that ES-BCR has occurred or indicates treatment failure.

The rate of rise in PSA level should be measured from the point of the PSA nadir. Patients whose velocity of increase in PSA rises above an indicator level can be characterized as undergoing early stage biochemical recurrence. The rate indicators can be obtained, evaluated, or determined by using statistical analyses including univariate logistic regression and receiver-operating characteristic (ROC) analysis, bivariate analysis or multivariate analysis or other appropriate statistical methods to obtain indicator values that provide good discrimination between patient subpopulations having stable disease and ES-BCR.

As discussed further below, the rate of rise in PSA levels over time is a good indicator of whether the patient has ES-BCR or stable disease. In addition, a rate indicator such as the second consecutive rise may be used as an indicator of whether the patient has ES-BCR or stable disease. The velocity of change in [PSA] indicator or the second consecutive rise indicator may be any amount between 0.2 and 2.5 pg/mL/month, preferably 0.2, 0.3, 0.4., 0.5, 0.6, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6. 2.7, 2.8, 2.9, 3.0, 3.2., 3.4, 3.6, 3.8, or 4.0 pg/mL/month, and more preferably 0.4, 0.5, 0.6, 0.7, 0.8, 0.9, 1.0 or 2.0 pg/mL per month, or about any of those amounts. As another example, the slope of Ln [PSA] vs. time indicator may be above about any level between 0.015 to 0.0425, preferably 0.015, .0175, 0.020, 0.0225, 0.025, 0.0275, 0.030, 0.0325, 0.035, 0.0375, 0.040, 0.0425, or 0.045, and more preferably 0.03.

In addition, in one embodiment a doubling time indicator of whether a patient has ES-BCR may be any number of days between 400-800 days, more preferably 400, 425, 450, 475, 500, 525, 550, 575, 600, 625, 650, 675, 700, 725, 750, 775 or 800 days, and most preferably 550 days. When the PSA rate value does not exceed the PSA rate indicator, the determination is made that the disease is stable; if the PSA rate value is equal to or exceeds the PSA rate indicator, ES-BCR is detected. In the case of a doubling time indicator, ES-BCR will be determined if the PSA doubling time in days is equal to or less than the doubling time indicator. For the doubling time value and indicator, the doubling time value will be determined to exceed the doubling time indicator if the doubling time value is less than the doubling time indicator.

In other embodiments, the maximum observed PSA indicator and slope of Ln [PSA] vs. time indicator are used in combination to determine whether a patient has stable disease or ES-BCR. For example, the method may further comprise c) determining that that the PSA level is above a PSA indicator of 15 pg/mL and that the slope of Ln [PSA] vs. time value is above a slope of Ln [PSA] vs. time indicator of about 0.03 in order to detect ES-BCR. On the other hand, if the PSA level is less than 15 pg/mL or the rate of rise in PSA level is below about 0.03, stable disease is detected. In this example, ES-BCR is determined if both the rate of increase in PSA level is equal to or exceeds the rate indicator, and the observed PSA value is equal to or exceeds the maximum observed PSA indicator.

In another aspect, the invention is a method of detecting whether a patient has fast, medium or slow early stage biochemical recurrence (ES-BCR), comprising
a) obtaining a serial set of blood serum samples from a patient after therapy for prostate cancer;
b) measuring the PSA level in each sample using a PSA assay having a functional sensitivity of about 0.5 pg/mL;
c) determining a PSA rate value;
d) determining that the PSA rate value is equal to or less than a PSA rate indicator, thereby detecting ES-BCR; and
e) classifying ES-BCR as rapid, medium, or slow based on the PSA rate indicator.

Patients whose PSA doubling time value is equal to or exceeds the rate threshold may be classified as having fast, medium or slow early stage biochemical recurrence (ES-BCR) based on doubling time. As an example, in some embodiments, a doubling time equal to or less than about ten months indicates fast or rapid recurrence; a doubling time of more than about ten months up to equal to or about 24 months indicates medium ES-BCR, and a doubling time of more than about 24 months indicates slow recurrence.

In another aspect, the invention is a method of detecting whether a patient has fast, medium or slow early stage biochemical recurrence (ES-BCR), comprising
a) obtaining a serial set of blood serum samples from a patient after therapy for prostate cancer;
b) measuring the PSA level in each sample using a PSA assay having a functional sensitivity of about 0.5 pg/mL;
c) determining the doubling time value and the maximum observed PSA value;
d) determining that the doubling time is equal to or less than a doubling time indicator, thereby detecting ES-BCR; and
e) classifying ES-BCR as rapid, medium, or slow based on the doubling time and maximum observed PSA.

In other aspects, patients whose PSA doubling time value is equal to or exceeds the doubling time threshold may be classified into four subclasses of ES-BCR based on doubling time and/or maximum observed PSA. Type 1 recurring patients have a doubling time of less than 150 days. Type 2 recurring patients have a doubling times between 150-400 days, Type 3 recurring patients and type 4 recurring patients have PSA doubling times greater than 400 days. For Type 3 patients, the maximum observed PSA exceeds 200 pg/mL, while Type 4 patients have maximum observed PSA values which do not exceed 200 pg/mL for an extended time of longer than 400 days.

In another aspect, the invention is a method of detecting if a patient has early stage biochemical recurrence (ES-BCR) after salvage therapy for prostate cancer, comprising
a) obtaining a samples from the patient after salvage therapy;
b) measuring the PSA level in the sample using a PSA assay having a functional sensitivity of about 0.5 pg/mL;
c) using the PSA level from one or more samples to determine a PSA value;
wherein ES-BCR is detected if the PSA value exceeds a PSA indicator and stable disease is detected if the PSA value does not exceed the PSA indicator.

In another aspect the invention is a kit comprising a nucleic acid-anti-PSA conjugate suitable for performing a sandwich immunoassay for PSA using PCR signal detection, wherein the assay has a detection limit at least as low as 0.2 pg/mL and a functional sensitivity at least as low as 0.5 pg/mL. The kit may further comprise software for determining one or more PSA values.

In another aspect, the invention is a label comprising a description of a method of detecting whether a patient has early stage biochemical relapse (ES-BCR), comprising
a) obtaining a sample from a patient after therapy for prostate cancer;
b) measuring the PSA level in the sample using a PSA assay having a functional sensitivity less than 1 pg/mL;
c) using the PSA level from one or more samples to determine a PSA value;
wherein ES-BCR is detected if the PSA value is equal to or exceeds a PSA indicator and stable disease is detected if the PSA value does not exceed the PSA indicator.

### EXAMPLES

Clinical studies using a higher sensitivity assay for total PSA (tPSA) showed that biochemical recurrence can be detected earlier by monitoring changes in serum PSA using the higher sensitivity assays. In contrast, the functional sensitivity of previously reported conventional assays is limited and cannot reliably report PSA levels less than around 0.01 ng/mL (10 pg/mL). Thus, use of high sensitivity [PSA] assays provides more reliable, early detection of BCR.

### Example 1: Nucleic Acid Detection Immunoassay (NADIA® assay) for the detection of very low levels of Prostate Specific Antigen (PSA)

Total PSA (tPSA) in serum samples was measured using a nucleic acid detection immunoassay (NADIA® assay) having a functional sensitivity of 0.5 pg/mL. See Clin Chem 53(6) Suppl., 2007, #C-15. The NADIA® assay is performed in sandwich immunoassay format.

Two antibodies directed to different epitopes on PSA were employed in an assay designed to detect pg/mL levels of PSA in patient samples from men who have undergone radical prostatectomy.

### Example 1A: Production of Signal Nucleic Acid-Anti-PSA Conjugate

The first antibody is conjugated (chemically linked) to an oligonucleotide of 60 bases as described by Jablonski and Adams in IVD Technology, November 2006. This reporter antibody is then diluted to approximately 10-30 picomolar (pM) concentration in a buffered diluent containing bovine serum albumin (BSA) and a surfactant to decrease nonspecific binding at a pH range of 7.0 - 7.5.

### Example 1B: Production of Capture Nucleic Acid-Anti-PSA Conjugate

The second antibody is immobilized on a para-magnetic particle of approximately 1 micron in diameter. The capture antibody has biotin chemically attached to it, using EZ-Link Sulfo-NHS-LC-Biotin (Sulfosuccinimidyl-6-(biotinamido) hexanoate, Catalog Number 21335 as supplied by Pierce using methods described in their catalog, and is subsequently bound to the para-magnetic particle through a streptavidin linker that has been attached to the magnetic particle by the manufacturer, Seradyn (Catalog Number 3015-2104).

### Example 1C: Conditions for NADIA® Assays

75 microliters (µl) of reporter antibody is allowed to react with 20 µl of patient serum sample for two hours at room temperature. In a heterogeneous format, the capture antibody, immobilized on the para-magnetic particles, is then added to the reporter antibody and sample solution. This mixture is allowed to react for 30 minutes with mild agitation to keep the para-magnetic particles in suspension.

At the end of this incubation the particles are separated magnetically from the remaining solution which is carefully removed leaving the magnetic particles on the side of the well. The magnetic particles are then washed 3-5 times removing non-bound reporter antibody. This solution is buffered at neutral pH containing a surfactant such as Tween 20. The result is a washed particle containing only PSA, if present, sandwiched by a capture antibody and a reporter antibody labeled with DNA.

PCR reagent containing complementary primers to the DNA and Taq polymerase is then added to the washed para-magnetic particles and real time PCR is performed. This PCR amplification step uses standard commercially available reagents. In the presence of an immune-complex, which contains DNA bound to the reporter antibody, amplification of the DNA template occurs.

The unknown sample is then read from a standard curve generated from calibrators of known tPSA concentration, 5, 25 and 100 pg/mL. Additionally each 96 well plate contains controls at 0.0, 10.0, and 80.0 pg/mL of PSA further ensuring the PCR amplification step is under proper control for each plate run.

As described in Jablonski and Adams in IVD Technology, November 2006, the assay can also be run in a homogenous format. For example, a first anti-PSA monoclonal antibody was labeled with an oligonucleotide sequence (a), and the second antibody was conjugated to oligonucleotide sequence (b) or (c). Oligonucleotide sequence (a) was complementary to the sequences (b) and (c), for the last 9 and 15 bases, respectively, at the 3' ends. The conjugate pair was diluted to 10-100 pmol in 10 mmol Tris (pH 8.0) containing 0.1% bovine serum albumin (BSA) and combined in the presence of PSA for 2 hours. The solution was then diluted with Tris/BSA to reduce the bulk conjugate concentration to below 1 pmol and was held at 52°C for 1 minute to fully melt unbound conjugate. PCR reagent mixture, containing Taq polymerase and downstream primers, was added, and the reaction was sealed. The temperature was lowered to 23°C to fully hybridize the DNA strands associated with the immune complex and to initiate the first chain extension. Free MAb-DNA cannot hybridize to the same degree in the time frame of the first extension in dilute solution, and cannot participate in subsequent exponential amplification. The overlapping DNA labels that were associated with the PSA immune complex were extended for 5 minutes, and completed by increasing the temperature to 85°C over 3 minutes. Real-time PCR amplification of the formed template was begun immediately, destroying the immune complex, which was no longer needed. The sensitivity of the assay was determined to be about 100 fg/mL.

To demonstrate the performance of the NADIA® PSA assay, IMD obtained patient samples from the Lab of Eleftherios Diamandis M.D. Ph.D. (University Health Network and Toronto Medical Laboratories, Toronto, ON, Canada). These samples included 42 patients which were previously characterized by Dr. Diamandis as stable and 43 patients with rising PSA values which he classified as having a biochemical recurrence. The samples were obtained post prostatectomy and were included if their PSA values post surgery dropped below 100 pg/mL. A biochemical recurrence was defined using several criteria and were based on time point values obtained during the course of the study. See Yu, He; Diamandis, Eleftherios, P.Wong, Pui-Yuen; Nam, Robert; Trachtenberg, John "Detection of Prostate Cancer Relapse with Prostate Specific Antigen Monitoring at Levels of .001 to 0.1 ug/L" J. Urology 157:913-18 (1997).

The NADIA® PSA assay was sensitive enough to precisely distinguish tPSA values in all female samples and the lowest observed values in the samples from the male population in the retrospective clinical study from background values.

### Example 2: Retrospective Study to Evaluate Indicators of Disease Outcome

NADIA® assays were used to measure tPSA levels in serial serum samples from prostate cancer patients following radical prostatectomy. The results were compared to earlier measurements of the PSA levels in the serum samples using a research assay based on an immunofluorometric (IFM) assay. Vassilikos et al., Clin. Biochem. 33: 115-123 (2000). The NADIA® assay results were then analyzed to determine concordance with the patient's clinical outcome.

### Samples

Serum samples (N=435) stored following a previously published study (J Urol 157:913-8, 1997) were used in this study. The samples were collected in 1993 and 1994, PSA levels were measured using the Abbott Laboratories IMx assay, and the samples were stored frozen at -40°C. The samples were also used in the study by Vassilikos et al. where the IFM assay was used to determine tPSA levels. The IFM assay is further described in Clin Chem 39:2108-14, 1993. The serum samples used in the Vassilikos et al. study were obtained from 85 patients who had baseline tPSA <100pg/mL post-RP (measured using the IMx assay), and who each had more than 3 serial samples taken post-RP (mean 5.0, median 5, range 3-6). Median (range) age was 63 years (49-73), pre-RP tPSA was 7.1ng/mL (0.1-49.0), Gleason score was 7 (5-9) and % tumor involvement was 25% (1-90%). Clinical stages were T1a-c (16), T2a-b (35) and unknown (33). 4 patients received pre-RP therapy (hormones=1; radiotherapy=2). In the Journal of Urology study, the serum samples for which tPSA values were originally determined with the Abbott IMx assay were re-analyzed by the IFM method and showed no significant differences compared to original values. The Journal of Urology article defined BCR as ≥2 successive tPSA increases reaching ≥100pg/mL, with relapse backdated to the first tPSA increase.

Serum samples from post-radical prostatectomy (RP) patients were included in the NADIA® assay PSA study if their PSA levels after a RP were below the detectable limit using currently FDA approved conventional PSA assays. Many of the conventional assays report that a patient has a zero or <0.1 ng/mL (<100 pg/mL) value post surgery. The NADIA® PSA assay can detect approximately a 200 fold lower level of PSA than the FDA approved PSA assays. Therefore, use of a higher sensitivity PSA assay permitted for the first time the measurement of the true level of PSA in post prostatectomy patients. The more sensitive and precise measurement of PSA levels allowed placement of patients into two groups-stable disease and early stage biochemical relapse.

### Descriptive Statistics for Patients in the Study

Seven patients were prospectively excluded from this analysis, because no NADIA® assay data were available or no surgery data was available. The final number of patients included in this study was eighty-five (85). Measurements of [PSA] (pg/mL) obtained by time of sampling for each patient included in the study are shown in Table 1, below.

| Patient ID | Recurrence (1=Yes, 0=No) | Days Post-Surgery | NADiA pg/ml PSA |
|---|---|---|---|
| 11 | 1 | 970 | 4.23 |
| | 1 | 1285 | 42.37 |
| | 1 | 1517 | 1255.62 |
| | 1 | 1708 | 2680.00 |
| 28 | 1 | 229 | 30.79 |
| | 1 | 550 | 109.86 |
| | 1 | 915 | 350.69 |
| | 1 | 1364 | 319.66 |
| | 1 | 1721 | 502.86 |
| 31 | 1 | 452 | 88.02 |
| | 1 | 660 | 159.86 |
| | 1 | 807 | 156.12 |
| | 1 | 2067 | 1859.00 |
| | 1 | 2431 | 2008.00 |
| 38 | 1 | 112 | 5.43 |
| | 1 | 224 | 17.10 |
| | 1 | 329 | 58.69 |
| | 1 | 763 | 189.08 |
| | 1 | 1444 | 883.84 |
| | 1 | 1666 | 1322.65 |
| 41 | 1 | 375 | 6.35 |
| | 1 | 508 | 10.41 |
| | 1 | 882 | 15.08 |
| | 1 | 1069 | 20.68 |
| | 1 | 1264 | 23.65 |
| | 1 | 1701 | 73.83 |
| 60 | 1 | 891 | 9.38 |
| | 1 | 1031 | 5.76 |
| | 1 | 1459 | 11.21 |
| | 1 | 1859 | 18.17 |
| | 1 | 2202 | 21.62 |
| | 1 | | |
| 64 | 1 | 460 | 44.30 |
| | 1 | 845 | 102.10 |
| | 1 | 1036 | 132.20 |
| | 1 | 2224 | 278.80 |
| 65 | 1 | 644 | 18.38 |
| | 1 | 806 | 25.68 |
| | 1 | 1565 | 114.02 |
| | 1 | 2011 | 216.38 |
| | 1 | 2150 | 278.46 |
| | 1 | 2312 | 388.57 |
| 79 | 1 | 938 | 147.10 |
| | 1 | 1281 | 155.80 |
| | 1 | 1366 | 193.70 |
| | 1 | 1557 | 197.80 |
| | 1 | 1731 | 271.80 |
| | 1 | 1974 | 2357.00 |
| 87 | 1 | 583 | 10.50 |
| | 1 | 751 | 9.69 |
| | 1 | 1081 | 17.63 |
| | 1 | 1458 | 35.58 |
| | 1 | 2192 | 105.97 |
| 89 | 1 | 424 | 97.80 |
| | 1 | 772 | 143.25 |
| | 1 | 857 | 221.32 |
| | 1 | 998 | 330.06 |
| | 1 | | 752.68 |
| 92 | 1 | 155 | 41.87 |
| | 1 | 301 | 54.52 |
| | 1 | 429 | 119.80 |
| | 1 | 513 | 153.72 |
| | 1 | 1785 | 1406.41 |
| 97 | 1 | 557 | 75.88 |
| | 1 | 698 | 455.62 |
| | 1 | 1264 | 542.54 |
| | 1 | 1672 | 726.70 |
| 103 | 1 | 716 | 29.12 |
| | 1 | 1243 | 6.45 |
| | 1 | 1621 | 65.48 |
| | 1 | 1781 | 164.06 |
| 105 | 1 | 655 | 10.52 |
| | 1 | 879 | 23.36 |
| | 1 | 1863 | 295.16 |
| | 1 | 2226 | 399.07 |
| 108 | 1 | 385 | 56.15 |
| | 1 | 887 | 306.78 |
| | 1 | 1224 | 378.35 |
| | 1 | 1586 | 661.77 |
| 113 | 1 | 540 | 4.57 |
| | 1 | 928 | 8.66 |
| | 1 | 1320 | 18.69 |
| | 1 | 1730 | 49.04 |
| | 1 | 2258 | 78.79 |
| 124 | 1 | 275 | 167.90 |
| | 1 | 631 | 331.40 |
| | 1 | 716 | 636.40 |
| | 1 | 1974 | 1782.00 |
| 136 | 1 | 81 | 9.70 |
| | 1 | 340 | 72.74 |
| | 1 | 515 | 184.14 |
| | 1 | 1757 | 647.86 |
| 151 | 1 | 188 | 39.13 |
| | 1 | 432 | 62.72 |
| | 1 | 830 | 169.00 |
| | 1 | 1061 | 382.34 |
| 160 | 1 | 248 | 10.84 |
| | 1 | 346 | 6.63 |
| | 1 | 528 | 24.15 |
| | 1 | 794 | 67.76 |
| | 1 | 976 | 122.64 |
| | 1 | 1354 | 228.00 |
| 177 | 1 | 1196 | 0.39 |
| | 1 | 1375 | 20.17 |
| | 1 | 1674 | 1.60 |
| | 1 | 2193 | 52.22 |
| | 1 | 2204 | 1.37 |
| 179 | 1 | 863 | 8.87 |
| | 1 | 1236 | 16.11 |
| | 1 | 1635 | 35.70 |
| | 1 | 2006 | 40.80 |
| | 1 | 2335 | 57.90 |
| 183 | 1 | 15 | 13.51 |
| | 1 | 218 | 77.75 |
| | 1 | 1041 | 255.50 |
| | 1 | 1375 | 520.09 |
| 184 | 1 | 281 | 6.59 |
| | 1 | 960 | 43.05 |
| | 1 | 1131 | 61.97 |
| | 1 | 1302 | 93.32 |
| | 1 | 1711 | 1722.20 |
| 197 | 1 | 490 | 42.80 |
| | 1 | 905 | 129.10 |
| | 1 | 1329 | 446.10 |
| | 1 | 1476 | 357.10 |
| | 1 | 1813 | 1585.30 |
| 214 | 1 | 184 | 20.66 |
| | 1 | 310 | 53.05 |
| | 1 | 1257 | 108.69 |
| | 1 | 1677 | 178.18 |
| | 1 | 2039 | 248.77 |
| 230 | 1 | 48 | 8.13 |
| | 1 | 138 | 10.36 |
| | 1 | 230 | 9.61 |
| | 1 | 671 | 38.85 |
| | 1 | 1588 | 201.80 |
| 242 | 1 | 128 | 8.69 |
| | 1 | 285 | 12.78 |
| | 1 | 582 | 82.14 |
| | 1 | 1163 | 178.67 |
| | 1 | 1541 | 277.47 |
| 261 | 1 | 47 | 13.21 |
| | 1 | 608 | 2227.87 |
| | 1 | 720 | 3267.70 |
| | 1 | 1026 | 60.10 |
| | 1 | 1132 | 241.10 |
| | 1 | 1385 | 2920.06 |
| 262 | 1 | 722 | 22.47 |
| | 1 | 1114 | 77.27 |
| | 1 | 1488 | 612.22 |
| | 1 | 1688 | 217.07 |
| | 1 | 1849 | 171.23 |
| 282 | 1 | 147 | 31.72 |
| | 1 | 793 | 171.80 |
| | 1 | 1165 | 299.61 |
| | 1 | 1362 | 678.51 |
| 300 | 1 | 48 | 4.70 |
| | 1 | 192 | 34.70 |
| | 1 | 350 | 108.80 |
| | 1 | 445 | 222.98 |
| | 1 | 592 | 267.07 |
| | 1 | 864 | 578.09 |
| 301 | 1 | 112 | 1.60 |
| | 1 | 265 | 3.12 |
| | 1 | 623 | 14.22 |
| | 1 | 833 | 27.30 |
| 302 | 1 | 54 | 6.48 |
| | 1 | 122 | 41.39 |
| | 1 | 410 | 528.84 |
| | 1 | 577 | 805.97 |
| | 1 | 748 | 941.12 |
| | 1 | 921 | 1302.18 |
| 303 | 1 | 86 | 3.45 |
| | 1 | 385 | 5.30 |
| | 1 | 545 | 14.10 |
| | 1 | 748 | 13.99 |
| | 1 | 1031 | 43.80 |
| | 1 | 1437 | 78.12 |
| 308 | 1 | 87 | 4.60 |
| | 1 | 177 | 19.93 |
| | 1 | 545 | 93.62 |
| | 1 | 744 | 196.28 |
| | 1 | 1028 | 295.98 |
| | 1 | 1210 | 484.54 |
| 309 | 1 | 60 | 1.27 |
| | 1 | 346 | 1.84 |
| | 1 | 756 | 2.02 |
| | 1 | 1188 | 81.39 |
| 312 | 1 | 188 | 35.89 |
| | 1 | 261 | 26.73 |
| | 1 | 391 | 258.85 |
| | 1 | 572 | 9338.12 |
| | 1 | 678 | 13316.08 |
| 322 | 1 | 155 | 4.41 |
| | 1 | 597 | 43.57 |
| | 1 | 839 | 87.82 |
| | 1 | 1128 | 180.12 |
| | 1 | 1241 | 255.53 |
| | 1 | 1601 | 315.70 |
| 325 | 1 | 101 | 1.36 |
| | 1 | 224 | 1.65 |
| | 1 | 686 | 5.59 |
| | 1 | 866 | 9.33 |
| | 1 | 1112 | 15.13 |
| | 1 | 1474 | 30.93 |
| 337 | 1 | 110 | 3.52 |
| | 1 | 482 | 1.31 |
| | 1 | 580 | 14.20 |
| | 1 | 671 | 69.91 |
| | 1 | 881 | 230.07 |
| | 1 | 1255 | 348.08 |
| 340 | 1 | 52 | 58.17 |
| | 1 | 71 | 79.29 |
| | 1 | 113 | 149.15 |
| | 1 | 393 | 476.20 |
| | 1 | 505 | 568.08 |
| | 1 | 1149 | 11857.37 |
| 29 | 0 | 108 | 5.15 |
| | 0 | 276 | 3.59 |
| | 0 | 473 | 7.85 |
| | 0 | 646 | 3.68 |
| | 0 | 1718 | 1.65 |
| 37 | 0 | 947 | 2.43 |
| | 0 | 1107 | 1.37 |
| | 0 | 1275 | 3.28 |
| | 0 | 1808 | 2.42 |
| | 0 | 2494 | 3.93 |
| 81 | 0 | 368 | 3.68 |
| | 0 | 712 | 2.49 |
| | 0 | 1084 | 4.37 |
| | 0 | 1516 | 2.03 |
| | 0 | 1716 | 3.38 |
| 82 | 0 | 755 | 9.47 |
| | 0 | 958 | 4.29 |
| | 0 | 1128 | 3.99 |
| | 0 | 1394 | 2.98 |
| | 0 | 2185 | 1.91 |
| 86 | 0 | 492 | 2.73 |
| | 0 | 667 | 2.22 |
| | 0 | 858 | 3.41 |
| | 0 | 1031 | 2.60 |
| | 0 | 1545 | 2.91 |
| 100 | 0 | 1288 | 1.42 |
| | 0 | 1652 | 3.35 |
| | 0 | 2030 | 1.99 |
| | 0 | 2770 | 1.20 |
| | 0 | 3133 | 1.38 |
| 120 | 0 | 638 | 2.48 |
| | 0 | 806 | 2.18 |
| | 0 | 977 | 0.82 |
| | 0 | 1150 | 3.52 |
| | 0 | 1536 | 1.65 |
| 126 | 0 | 585 | 6.34 |
| | 0 | 892 | 1.34 |
| | 0 | 1477 | 0.79 |
| | 0 | 1896 | 3.39 |
| | 0 | 2166 | 3.89 |
| | 0 | 2273 | 1.03 |
| 128 | 0 | 212 | 8.11 |
| | 0 | 331 | 3.56 |
| | 0 | 513 | 1.60 |
| | 0 | 605 | 2.67 |
| | 0 | 1788 | 2.31 |
| 137 | 0 | 202 | 4.19 |
| | 0 | 356 | 1.44 |
| | 0 | 541 | 1.09 |
| | 0 | 723 | 1.88 |
| | 0 | 1078 | 1.46 |
| | 0 | 1416 | 0.87 |
| 144 | 0 | 203 | 3.13 |
| | 0 | 359 | 1.94 |
| | 0 | 532 | 5.09 |
| | 0 | 994 | 3.89 |
| | 0 | 1392 | 5.47 |
| | 0 | 1815 | 1.73 |
| 154 | 0 | 842 | 1.79 |
| | 0 | 1444 | 0.85 |
| | 0 | 1528 | 2.23 |
| | 0 | 1808 | 2.03 |
| | 0 | 2235 | 1.51 |
| | 0 | 2403 | |
| 164 | 0 | 315 | 5.77 |
| | 0 | 539 | 4.97 |
| | 0 | 1316 | 6.00 |
| | 0 | 1703 | 4.10 |
| | 0 | 1983 | 6.01 |
| 167 | 0 | 877 | 17.35 |
| | 0 | 1231 | 22.01 |
| | 0 | 1926 | 24.20 |
| | 0 | 2226 | 127.05 |
| 178 | 0 | 181 | 0.19 |
| | 0 | 251 | 0.15 |
| | 0 | 469 | 0.21 |
| | 0 | 1007 | 3.62 |
| | 0 | 1387 | 4.68 |
| | 0 | 1578 | 0.12 |
| 191 | 0 | 61 | 2.19 |
| | 0 | 256 | 1.36 |
| | 0 | 727 | 1.19 |
| | 0 | 987 | 6.27 |
| | 0 | 1385 | 1.17 |
| | 0 | 1687 | 2.72 |
| 193 | 0 | 61 | 5.56 |
| | 0 | 152 | 3.09 |
| | 0 | 277 | 6.43 |
| | 0 | 999 | 10.37 |
| | 0 | 1196 | 7.27 |
| 196 | 0 | 33 | 4.31 |
| | 0 | 537 | 1.97 |
| | 0 | 922 | 2.25 |
| | 0 | 1289 | 3.33 |
| | 0 | 1634 | 4.29 |
| 219 | 0 | 257 | 1.34 |
| | 0 | 700 | 7.71 |
| | 0 | 852 | 1.95 |
| | 0 | 1444 | 1.38 |
| 227 | 0 | 49 | 4.40 |
| | 0 | 235 | 4.13 |
| | 0 | 353 | 8.60 |
| | 0 | 446 | 25.08 |
| | 0 | 616 | 6.80 |
| | 0 | 790 | 8.00 |
| 231 | 0 | 1243 | 5.28 |
| | 0 | 1564 | 10.53 |
| | 0 | 1923 | 14.46 |
| | 0 | 2292 | 14.79 |
| | 0 | 2657 | 13.99 |
| 235 | 0 | 57 | 1.93 |
| | 0 | 87 | 4.76 |
| | 0 | 196 | 4.33 |
| | 0 | 415 | 6.34 |
| | 0 | 570 | 4.49 |
| | 0 | 967 | 4.05 |
| 244 | 0 | 299 | 2.10 |
| | 0 | 516 | 2.55 |
| | 0 | 760 | 3.27 |
| | 0 | 969 | 5.26 |
| | 0 | 1146 | 2.03 |
| | 0 | | 3.17 |
| 246 | 0 | 104 | 4.48 |
| | 0 | 229 | 11.95 |
| | 0 | 391 | 8.40 |
| | 0 | 761 | 4.13 |
| | 0 | 1104 | 3.64 |
| | 0 | 1498 | 5.24 |
| 254 | 0 | 118 | 1.23 |
| | 0 | 166 | 1.59 |
| | 0 | 811 | 3.11 |
| | 0 | 1154 | 2.58 |
| 255 | 0 | 1321 | 3.60 |
| | 0 | 1477 | 3.17 |
| | 0 | 1607 | 3.93 |
| | 0 | 1883 | 4.32 |
| | 0 | 2175 | 2.50 |
| | 0 | 2525 | 9.68 |
| 259 | 0 | 75 | 1.60 |
| | 0 | 173 | 2.44 |
| | 0 | 393 | 2.74 |
| | 0 | 581 | 2.14 |
| | 0 | 1042 | 1.90 |
| | 0 | 1526 | 2.89 |
| 265 | 0 | 175 | 5.01 |
| | 0 | 742 | 1.18 |
| | 0 | 1115 | 0.87 |
| | 0 | 1615 | 0.80 |
| 266 | 0 | 55 | 3.78 |
| | 0 | 191 | 3.90 |
| | 0 | 321 | 6.76 |
| | 0 | 697 | 5.05 |
| | 0 | 1035 | 4.87 |
| | 0 | 1480 | 14.39 |
| 280 | 0 | 428 | 2.08 |
| | 0 | 616 | 2.37 |
| | 0 | 990 | 0.71 |
| | 0 | 1401 | 1.02 |
| | 0 | 1813 | 2.20 |
| 285 | 0 | 220 | 1.01 |
| | 0 | 591 | 3.06 |
| | 0 | 955 | 0.98 |
| | 0 | 1147 | 1.27 |
| | 0 | 1343 | 0.81 |
| | 0 | 1493 | 3.47 |
| 290 | 0 | 91 | 1.38 |
| | 0 | 210 | 2.54 |
| | 0 | 478 | 3.73 |
| | 0 | 842 | 3.81 |
| | 0 | 1037 | 2.75 |
| | 0 | 1420 | 5.25 |
| 296 | 0 | 131 | 4.28 |
| | 0 | 552 | 3.06 |
| | 0 | 798 | 1.83 |
| | 0 | 976 | 3.34 |
| | 0 | 1178 | 1.01 |
| | 0 | 1464 | 1.23 |
| 305 | 0 | 55 | 2.39 |
| | 0 | 328 | 1.74 |
| | 0 | 738 | 2.66 |
| | 0 | 951 | 2.18 |
| | 0 | 1140 | 1.69 |
| | 0 | 1418 | 2.19 |
| 313 | 0 | 37 | 4.46 |
| | 0 | 95 | 3.86 |
| | 0 | 199 | 6.51 |
| | 0 | 472 | 7.71 |
| | 0 | 815 | 6.17 |
| | 0 | 1144 | 6.00 |
| 317 | 0 | 719 | 3.09 |
| | 0 | 930 | 0.76 |
| | 0 | 1094 | 1.01 |
| | 0 | 1315 | 0.89 |
| | 0 | 1749 | 1.18 |
| | 0 | 2543 | 3.58 |
| 321 | 0 | 91 | 0.92 |
| | 0 | 242 | 0.74 |
| | 0 | 641 | 0.70 |
| | 0 | 1005 | 1.13 |
| | 0 | 1440 | 1.77 |
| 326 | 0 | 24 | 2.40 |
| | 0 | 252 | 1.47 |
| | 0 | 426 | 1.49 |
| | 0 | 860 | 0.75 |
| | 0 | 1180 | 3.86 |
| | 0 | 1298 | 4.62 |
| 330 | 0 | 69 | 5.06 |
| | 0 | 524 | 5.64 |
| | 0 | 624 | 5.16 |
| | 0 | 820 | 6.80 |
| | 0 | 1016 | 7.71 |
| | 0 | 1234 | 6.56 |
| 336 | 0 | 75 | 1.66 |
| | 0 | 256 | 1.64 |
| | 0 | 599 | 2.45 |
| | 0 | 788 | 1.13 |
| | 0 | 958 | 1.07 |
| | 0 | 1313 | 1.61 |
| 341 | 0 | 58 | 19.33 |
| | 0 | 165 | 7.84 |
| | 0 | 382 | 4.49 |
| | 0 | 697 | 4.77 |
| | 0 | 1137 | 3.97 |
| | 0 | 1270 | 2.86 |
| 347 | 0 | 785 | 1.96 |
| | 0 | 1179 | 4.29 |
| | 0 | 1366 | 2.81 |
| | 0 | 1555 | 2.90 |
| | 0 | 1793 | 3.64 |
| | 0 | | |

Forty-three (43) were classified as recurring and forty-two (42) were classified as having stable disease based on the Diamandis research assay. Yu, et al., J. Urology 157:913-18 (1997). Clinicopathological variable descriptive statistics for the patient populations were obtained. Significance of differences in the clinical variables distribution between patients in recurring and stable disease classifications are summarized in Table 2 below (p< 0.05 indicated a significant difference in the distribution of the variable between the recurring population and the stable disease population.

**TABLE 2: Clinicopathological variables - significance of differences in distribution between recurring and stable disease patients**

| **Variable** | **N** | **p** |
|---|---|---|
| Age at diagnosis | 68 | 0.6117* |
| Stage | 51 | 0.3324** |
| Gleason Score | 66 | ***0.0276***** |
| Pre-op chemotherapy | 55 | 0.1611** |
| Treatment Type | 51 | 0.4216** |
| Margin involved | 61 | ***0.0006***** |
| Peri prostatic tissue invasion | 51 | ***0.0006***** |
| Capsular invasion | 62 | ***0.0181***** |
| Seminal vesicle invasion | 62 | 0.6216** |
| Bladder neck invasion | 51 | 0.7037** |
| Lymph node involved | 60 | n/a |
| Tumor volume | 56 | ***0.0008**** |

| | | |
|---|---|---|
| ** INilcoxon rank sum* ** *Chi-square* | | |

In the current study, eighty-four (98.8%) of the patients were evaluable for biochemical evaluation using NADIA® assays to measure tPSA, and 60-70% of them were evaluable clinicopathologically. Measuring tPSA using NADIA® showed that the median (range) nadir or first tPSA value post-RP was 4.1 pg/mL (0.2-167.9 pg/mL).

In addition, as shown in the table above of the significance of differences in the distribution of clinical variables between recurring and stable disease classifications: Gleason, Surgical margin, Peri-prostatic invasion, Capsular invasion, and Tumor volume all show significant differences between sub-populations and may be predictors of outcomes.

### Example 3: Evaluation of [PSAI Based Measurements as Indicator(s) of Disease Outcome

Analysis of the data collected for the sample set permitted evaluation of hypotheses that various PSA measurement indicators were predictive of disease outcome, and would be useful in monitoring patients following therapy for prostate cancer. These indicators included the following values based on NADIA® assay measurements of tPSA in serial samples from patients: tPSA doubling time (calculated only from patients for whom NADIA® assay PSA values were capable of exponential fitting); first post-prostatectomy level (the nadir value is not always the same as the first post-prostatectomy value); maximum tPSA level observed post-nadir (can be at any point in monitoring); ratio of maximum tPSA level to nadir (requires at least one value higher than the apparent nadir level at some time point after the nadir to indicate a possible recurrence); second consecutive increase pg/mL/month; rate of increase; number of doublings during the monitoring period; number of consecutive doublings during monitoring.

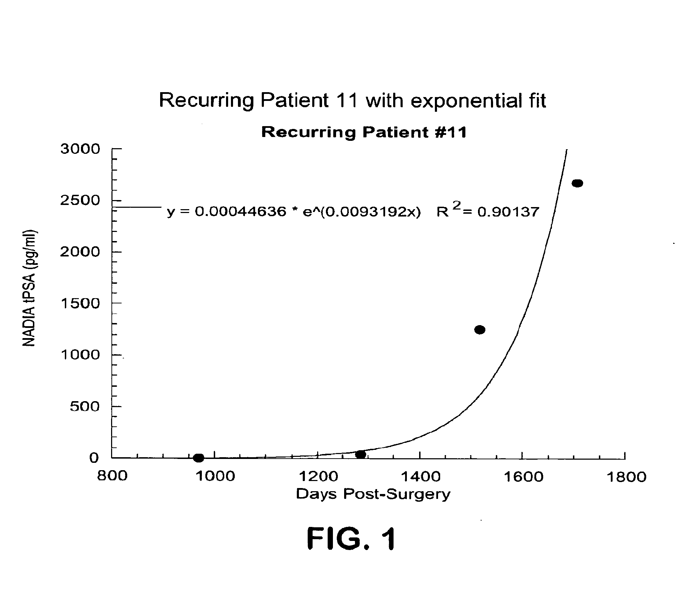

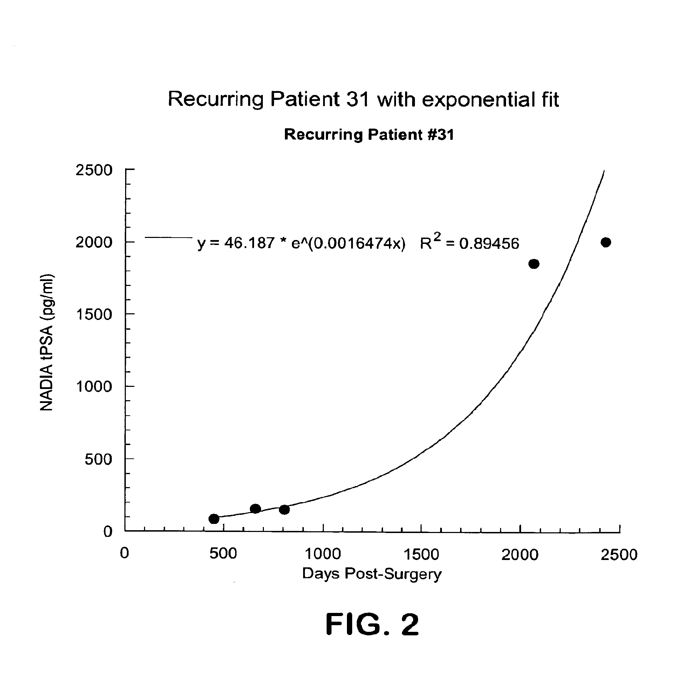

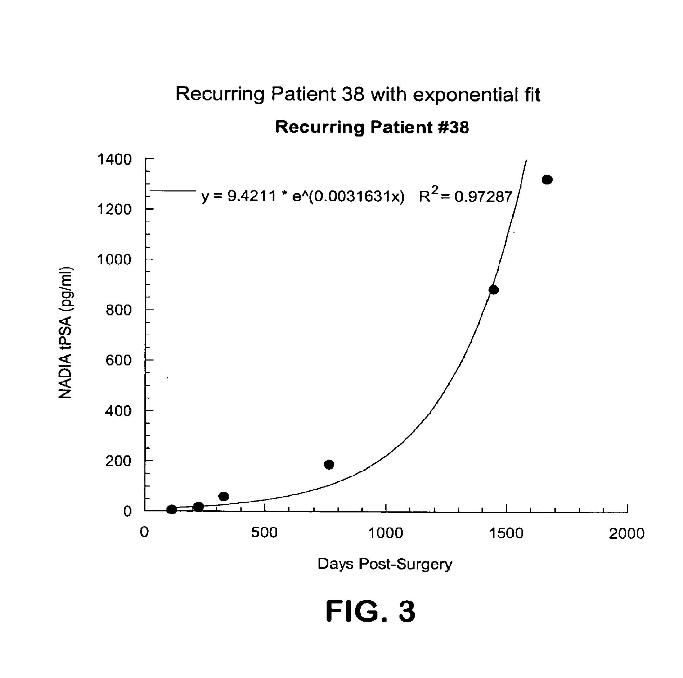

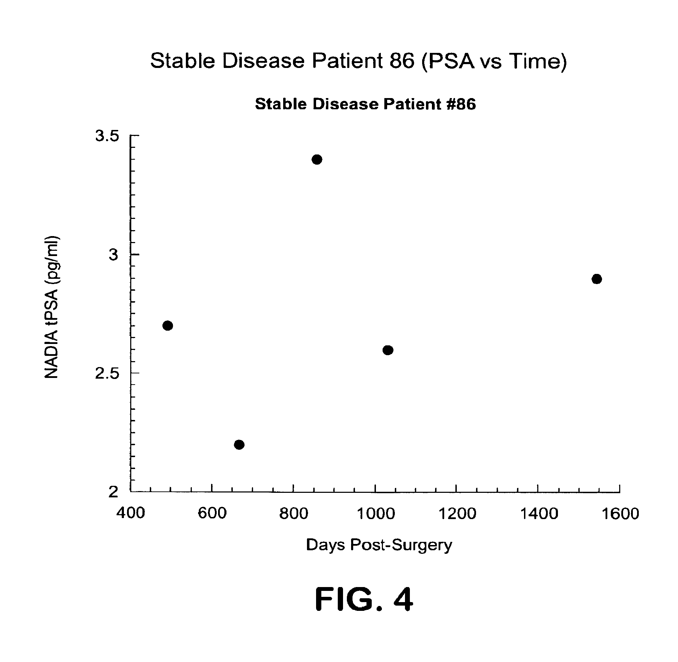

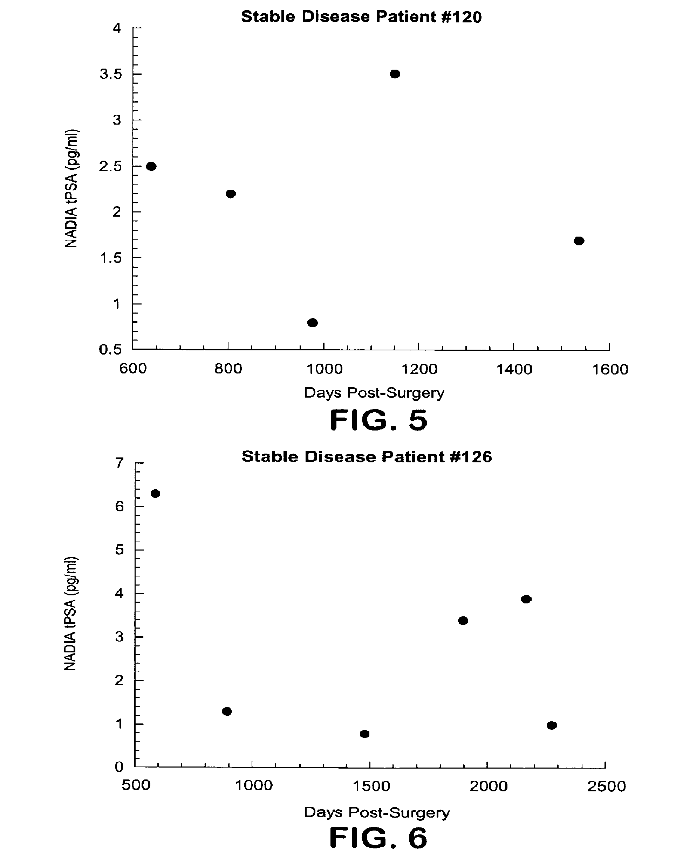

For each patient analyzed in this study, the tPSA (pg/mL) measured using NADIA® assays was plotted as a function of days post-surgery. For example, Figure 1 shows the plot of the NADIA® t[PSA] in pg/mL vs. days post radical prostatectomy for recurring patient number 11, with exponential fit. Figure 2 shows the plot of the NADIA® t[PSA] in pg/mL vs. days post radical prostatectomy for recurring patient number 31, with exponential fit. Figure 3 shows the plot of the NADIA® t[PSA] in pg/mL vs. days post radical prostatectomy for recurring patient number 38, with exponential fit. Figure 4 shows the plot of the NADIA® t[PSA] in pg/mL vs. days post radical prostatectomy for stable patient number 86, with exponential fit. Figure 5 shows the plot of the NADIA® t[PSA] in pg/mL vs. days post radical prostatectomy for stable patient number 120, with exponential fit. Figure 6 shows the plot of the NADIA® [PSA] in pg/mL vs. days post radical prostatectomy for stable patient number 126, with exponential fit.

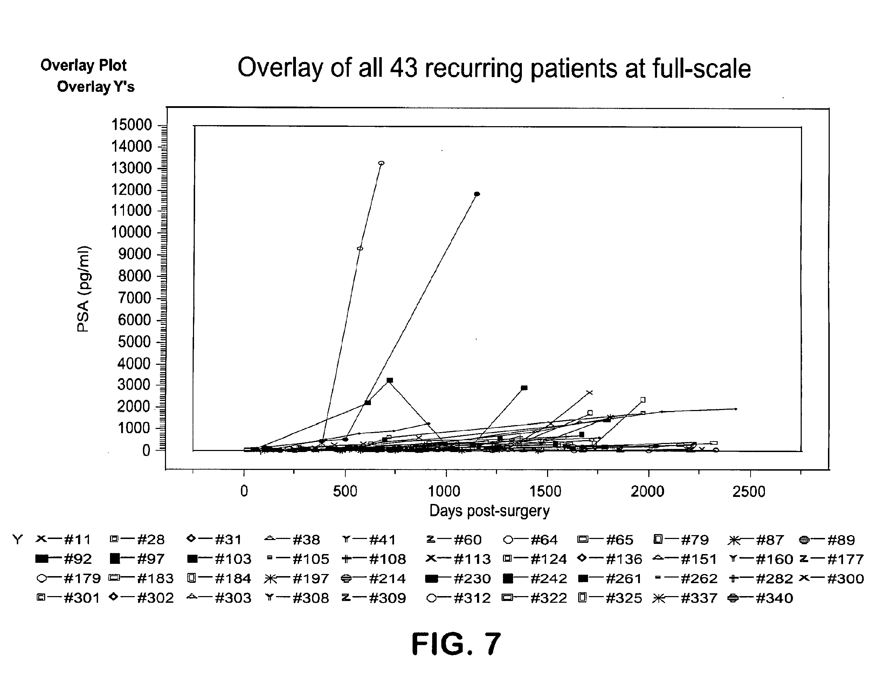

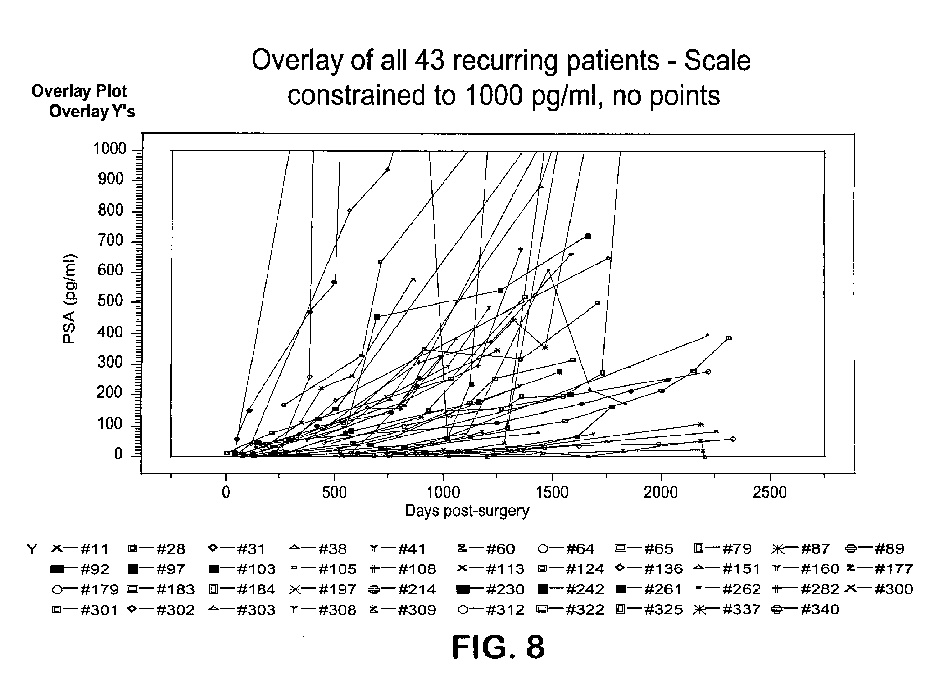

The plots for all patients were separated by whether patients fell into the Recurring category or Stable Disease category. Figure 7 shows the plots of the NADIA® t[PSA] in pg/mL vs. days post radical prostatectomy for all 43 recurring patients. Figure 8 shows an overlay plot of the NADIA® t[PSA] for 43 recurring patients vs time following prostatectomy, with range constrained to 1000 pg/ml, no points.

In the analysis of doubling time, the study excluded stable disease patients whose plots could not be fitted exponentially. Ten of the 42 stable disease patients were included in the doubling time analysis. For all other analyses (maximum observed PSA level, first post-prostatectomy PSA level, nadir PSA level, maximum observed PSA level/nadir level ratio, number of doublings, number of successive doublings, and 2^{nd} pg/mL/month rise) data from all 43 recurring and all 42 stable disease patients was utilized, *i.e*., no exclusions were made.

### Example 4: Analyses of potential indicators for disease outcome

An analysis of each possible PSA indicator (first post-prostatectomy PSA level, nadir PSA level, maximum observed PSA level, maximum observed PSA level/nadir level ratio, number of doublings, number of successive doublings, 2^{nd} pg/m./month rise, doubling time (where exponential fits were possible)) versus recurring or stable disease was performed to assess the relative utility of each outcome as a predictor of recurrence. Clinical classification of patients as stable or having disease recurrence was used as a reference outcome. The statistical tests used were the Wilcoxon rank sum test for continuous variables, and the Pearson chi-square test for categorical variables.

The analyses demonstrated that all of the calculated [PSA] parameters were significant predictors (Wilcoxon rank sum or Pearson chi-square p< 0.05) of clinical outcome (recurrence or stable disease). The maximum observed tPSA level, second consecutive increase pg/mL/month, and doubling time were the best at discriminating the patient sub-populations. The ratio of maximum PSA level to nadir level and the number of doublings also demonstrated fair discrimination.

The analysis for each of the [PSA] indicators is discussed below.

### Example 4A: Analysis of 1st Post-Prostatectomy Level vs. Patient Sub-Population (Recurrence or Stable Disease)

| **TABLE 4: Quantiles** | | | | | | | |
|---|---|---|---|---|---|---|---|
| Level | Minimum | 10% | 25% | Median | 75% | 90% | Maximum |
| 0 | 1.2 | 2.2 | 3.15 | 4.5 | 7.7 | 14.15 | 127 |
| 1 | 21.6 | 54.48 | 164 | 484.5 | 1406.4 | 2550.8 | 13316 |

| **TABLE 5: Means and Std Deviations** | | | | | | |
|---|---|---|---|---|---|---|
| Level | Number | Mean | Std Dev | Std Err MeanLower 95% | | Upper 95% |
| 0 | 40 | 8.96 | 19.67 | 3.11 | 2.67 | 15.3 |
| 1 | 43 | 1296.86 | 2648.59 | 403.91 | 481.75 | 2112.0 |

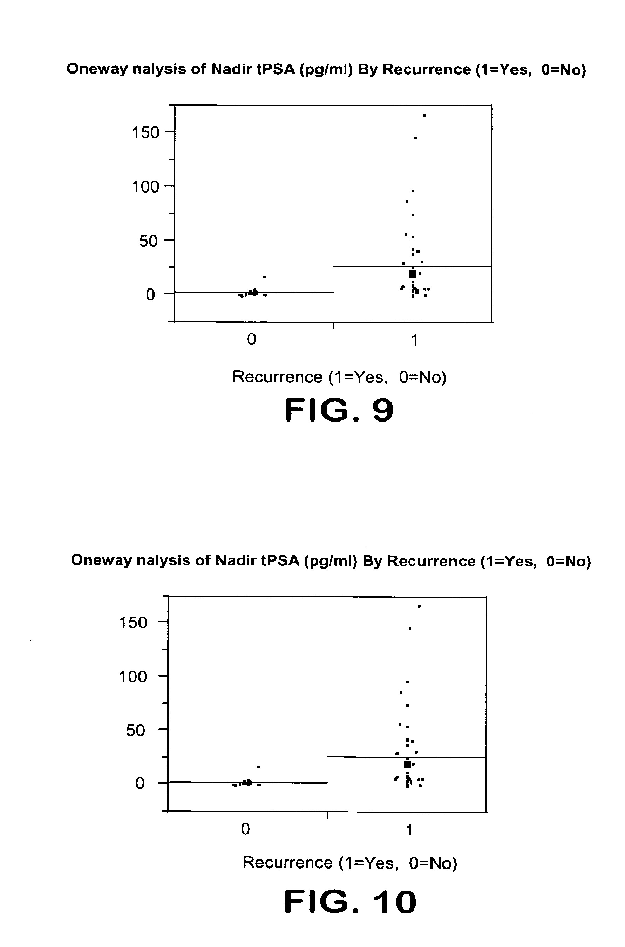

A plot of the first post-prostatectomy tPSA level vs. the patient sub-population (recurrence of prostate cancer (1) or with stable disease (0)) is shown in Figure 9. Quintiles for the stable disease group (0) and the recurrence group (1) are shown in Table 4. The means and standard deviations for the stable disease group (0) and the recurrence group (1) are shown in Table 5. According to the data analysis for the plot of the first post-prostatectomy tPSA level vs. the patient sub-population (recurrence or stable disease), this parameter significantly differentiates the two populations and is thus a predictor of outcomes. The mean +/- standard error of the mean (SEM) [PSA] for the stable group was 4.1 pg/mL +/-0.58, while the mean +/- SEM [PSA] for the group having recurrence was 28.2 +/- 5.72. The p was < 0.0001. However, the stable population overlaps the recurring population up to and beyond the median value.

### Example 4B: Analysis of Nadir TPSA Level vs. Patient Sub-Population (Recurrence or Stable Disease):

| **TABLE 6: Quantiles** | | | | | | | |
|---|---|---|---|---|---|---|---|
| Level | Minimum | 10% | 25% | Median | 75% | 90% | Maximum |
| 0 | 0.2 | 0.8 | 0.975 | 1.7 | 2.95 | 4.38 | 17.4 |
| 1 | 0.4 | 1.48 | 4.7 | 9.7 | 39.1 | 83.16 | 167.9 |

| **TABLE 7: Means and Std Deviations** | | | | | | |
|---|---|---|---|---|---|---|
| Level | Number | Mean | Std Dev | Std Err Mean | Lower 95% | Upper 95% |
| 0 | 42 | 2.3976 | 2.7038 | 0.4172 | 1.555 | 3.240 |
| 1 | 43 | 27.1605 | 37.7972 | 5.7640 | 15.528 | 38.793 |

A plot of the nadir t[PSA] level (pg/mL) vs. the patient sub-population (recurrence of prostate cancer (1) or with stable disease (0)) is shown in Figure 10. Quintiles for the stable disease group (0) and the recurrence group (1) are shown in Table 6. The means and standard deviations for the stable disease group (0) and the recurrence group (1) are shown in Table 7. According to the data analysis for the nadir [PSA] level, this parameter significantly differentiates the two populations and is thus a predictor of outcome. The mean +/- SEM nadir [PSA] for the stable group was 2.4 pg/mL +/- 0.42, while the mean +/- SEM nadir [PSA] for the group having recurrence was 27.2 +/- 5.8. The p was < 0.0001. However, the stable population overlaps the recurring population up to and beyond the median value.

### Example 4C: Analysis of Maximum Observed TPSA Level vs. Patient Sub-Population (Recurrence or Stable Disease):

| **TABLE 8: Quantiles** | | | | | | | |
|---|---|---|---|---|---|---|---|
| Level | Minimum | 10% | 25% | Median | 75% | 90% | Maximum |
| 0 | 1.2 | 2.2 | 3.15 | 4.5 | 7.7 | 14.15 | 127 |
| 1 | 21.6 | 54.48 | 164 | 484.5 | 1406.4 | 2550.8 | 13316 |

| **TABLE 9: Means and Std Deviations** | | | | | | |
|---|---|---|---|---|---|---|
| Level | Number | Mean | Std Dev | Std Err Mean | Lower 95% | Upper 95% |
| 0 | 40 | 8.96 | 19.67 | 3.11 | 2.67 | 15.3 |
| 1 | 43 | 1296.86 | 2648.59 | 403.91 | 481.75 | 2112.0 |

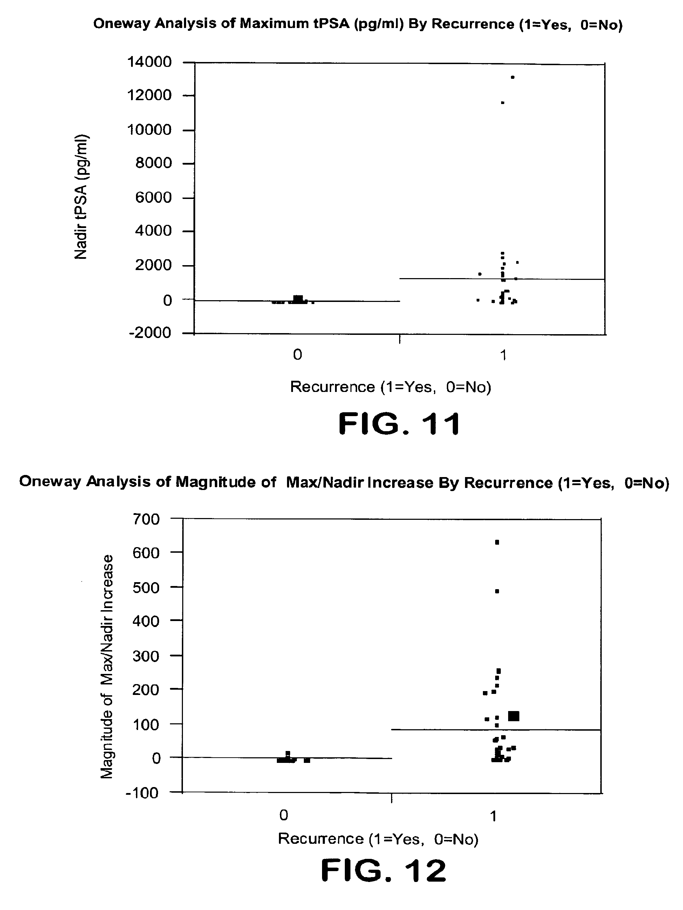

A plot of the maximum observed [PSA] level (pg/mL) vs. the patient sub-population (recurrence of prostate cancer (1) or with stable disease (0)) is shown in Figure 11. Quantiles for the stable disease group (0) and the recurrence group (1) are shown in Table 8. The means and standard deviations for the stable disease group (0) and the recurrence group (1) are shown in Table 9. Analysis of the maximum observed [PSA] level vs. the patient sub-population showed that the maximum tPSA level significantly differentiated the two populations of stable and recurring patients and was therefore a predictor of outcome. The mean +/- SEM [PSA] for the stable group was 9.0 pg/mL +/- 3.11, while the mean +/- SEM [PSA] for the group having recurrence was 1295.9 +/- 403.91. The p was < 0.0001. The stable population only overlaps the recurring population somewhere between 10 and 25% and was thus nicely discriminated. In this study there was only one stable disease patient with an observed PSA level above 15 pg/mL.

### Example 4D: Analysis of Maximum tPSA Level/NAdir Level vs. Patient Sub-Population (Recurrence or Stable Disease):

| **TABLE 10: Quantiles** | | | | | | | |
|---|---|---|---|---|---|---|---|
| Level | Minimum | 10% | 25% | Median | 75% | 90% | Maximum |
| 0 | 1.2 | 1.5 | 1.8 | 2.6 | 4.4 | 5.9 | 23.5 |
| 1 | 3.4 | 7.74 | 12 | 27.2 | 123 | 254.54 | 638.1 |

| **TABLE 11: Means and Std Deviations** | | | | | | |
|---|---|---|---|---|---|---|
| Level | Number | Mean | Std Dev | Std Err Mean | Lower 95% | Upper 95% |
| 0 | 39 | 3.6154 | 3.602 | 0.577 | 2.448 | 4.78 |
| 1 | 43 | 87.5372 | 133.004 | 20.283 | 46.605 | 128.47 |

A plot of the maximum [PSA] level (pg/mL)/nadir level [PSA] (pg/mL) vs. the patient sub-population (recurrence of prostate cancer (1) or with stable disease (0)) is shown in Figure 12. Quantiles for the stable disease group (0) and the recurrence group (1) are shown in Table 10. The means and standard deviations for the stable disease group (0) and the recurrence group (1) are shown in Table 11. Analysis of the maximum PSA level/nadir level PSA vs. patient sub-population showed that the ratio of the maximum PSA level to the nadir [PSA] significantly differentiates the two populations and is thus a predictor of outcome. The p was < 0.001. The mean +/- SEM for the stable population was 3.6 +/-0.6, while the mean +/- SEM for the recurring population was 87.5 +/- 20.3. However, the stable population overlaps the recurring population close to the median value.

### Example 4E: Analysis of 2nd Consecutive Increase pg/mL/month vs. Patient Sub-Population (Recurrence or Stable Disease):

| **TABLE 12: Quantiles** | | | | | | | |
|---|---|---|---|---|---|---|---|
| Level | Minimum | 10% | 25% | Median | 75% | 90% | Maximum |
| 0 | -0.73 | -0.195 | -0.085 | 0.015 | 0.175 | 0.332 | 5.4 |
| 1 | -140.7 | 1.64 | 4.7 | 7 | 20.1 | 117.36 | 1526.8 |

| **TABLE 13: Means and Std Deviations** | | | | | | |
|---|---|---|---|---|---|---|
| Level | Number | Mean | Std Dev | Std Err Mean | Lower 95% | Upper 95% |
| 0 | 42 | 0.1490 | 0.861 | 0.133 | -0.12 | 0.42 |
| 1 | 43 | 63.4930 | 241.163 | 36.777 | -10.73 | 137.71 |

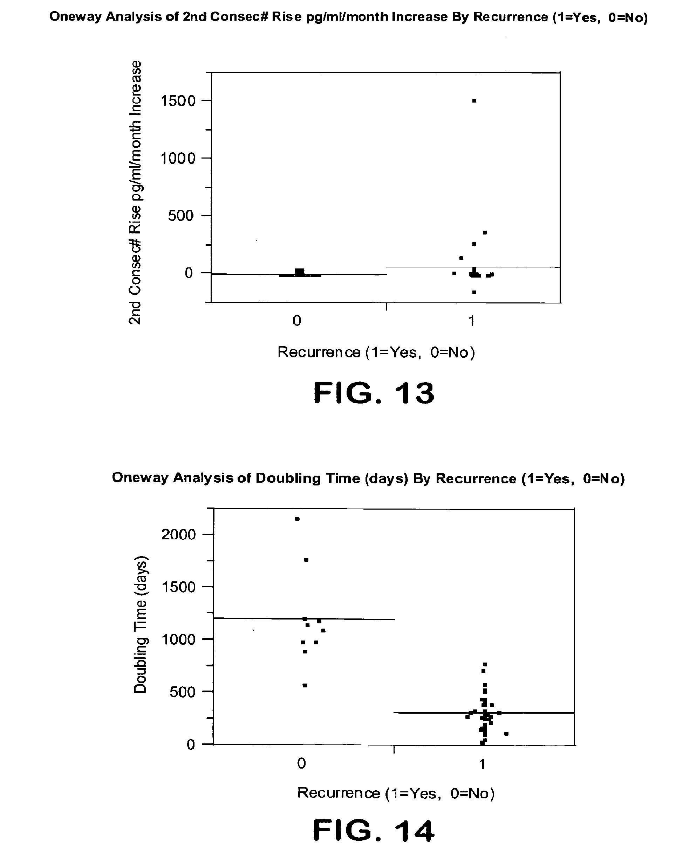

A plot of the second consecutive increase in [PSA] level (pg/mL/month) vs. the patient sub-population (recurrence of prostate cancer (1) or with stable disease (0) is shown in Figure 13. Quantiles for the stable disease group (0) and the recurrence group (1) are shown in Table 12. The means and standard deviations for the stable disease group (0) and the recurrence group (1) are shown in Table 13. The analysis for the second consecutive increase (pg/mL/month) showed that this parameter significantly differentiates the two populations and is thus a predictor of outcome. The mean +/- SEM second consecutive increase for the stable group was 0.15 pg/mL/month +/- 0.13, while the mean +/- SEM or the group having recurrence was 63.5 +/- 36.78. The p was < 0.0001. The stable population overlaps the recurring population approximately 25% and thus indicates a good discriminatory power.

### Example 4F: Analysis of Doubling Time (days) vs. Patient Sub-Population (Recurrence or Stable Disease):

| **TABLE 14: Quantiles** | | | | | | | |
|---|---|---|---|---|---|---|---|
| Level | Minimum | 10% | 25% | Median | 75% | 90% | Maximum |
| 0 | 577.6 | 611.04 | 970.65 | 1127.7 | 1356.325 | 2127.22 | |
| | | | | | | | 2166.1 |
| 1 | 49.2 | 127.26 | 203.9 | 291.9 | 407.7 | 544.54 | 796.7 |

| **TABLE 15: Means and Std Deviations** | | | | | | |
|---|---|---|---|---|---|---|
| Level | Number | Mean | Std Dev | Std Err Mean | Lower 95% | Upper 95% |
| 0 | 10 | 1207.99 | 451.736 | 142.85 | 884.84 | 1531.1 |
| 1 | 40 | 318.55 | 164.681 | 26.04 | 265.88 | 371.2 |

A plot of the doubling time data (days) vs. the patient sub-population (recurrence of prostate cancer (1) or with stable disease (0)) is shown in Figure 14. Quintiles for the stable disease group (0) and the recurrence group (1) are shown in Table 14. The means and standard deviations for the stable disease group (0) and the recurrence group (1) are shown in Table 15. Analysis of the data showed that the doubling time (days) significantly differentiates the two populations and is thus a predictor of outcome. The p was < 0.0001. The mean for the stable population was 1208 +/ 142.9, while the mean for the recurring population was 318.6 +/- 26.04. The stable population only overlaps the recurring population between 10 and 25% and is thus nicely discriminated.

### Additional categorization of patients based on doubling time observed using a PSA assay

Further analysis was undertaken to determine whether the doubling time could be used to discriminate between further subclasses of the recurring subpopulation of patients. The analysis of PSA doubling time permitted further sorting of patients into three groups, categorized by < 150 days (rapid recurrences), 150-400 days (medium recurrences), and > 400 days (slow recurrences). Rate was expected to reflect the rate of exponential growth, and therefore reflect the aggressiveness of the growth of the cancer.

Figures 15A-C show the overlay plots for recurring patients with doubling times of < 150 days, 150-400 days, or > 400 days, respectively. Figure 15A shows the overlay plots for recurring patients, of [PSA] pg/ml vs days post surgery, with doubling times of < 150 with range constrained to 1000 pg/mL

Figure 15B shows the overlay plots for recurring patients, of [PSA] pg/ml vs days post surgery with doubling times of 150-400 with range constrained to 1000 pg/mL

Figure 15C shows the overlay plots for recurring, of [PSA] pg/ml vs days post surgery patients with doubling times of >400 with range constrained to 1000 pg/Ml

The recurring patients can be divided into four classes, Group 1, doubling time of less than 150 days, Group 2, with doubling times between 150-400 days, Groups 3 and 4, which both had doubling times greater than 400 days. In Group 3 the maximum observed PSA exceeded 200 pg/mL, while in Group 4 the maximum observed PSA did not exceed 200 pg/mL.

Figures 16A-D shows the overlay plots for subclasses of recurring patients by doubling time, with ranges constrained to 1000 pg/mL, respectively. The recurring patients with doubling times of > 400 days have been further subdivided whether the maximum observed PSA is above or below 200 pg/mL.

Figure 16A shows the overlay plots for recurring patients with doubling time < 150 days of [PSA] pg/ml vs days post surgery.

Figure 16B shows the overlay plots for recurring patients with doubling time < 150-400 days of [PSA] pg/ml vs days post surgery.

Figure 16C shows the overlay plots for recurring patients with doubling time> 400 days, maximum [PSA] > 200 pg/mL vs days post surgery.

Figure 16D shows the overlay plots for recurring patients [PSA] pg/ml vs days post surgery.

Figure 17 shows the overlay plots of [PSA] pg/ml vs days post surgery that, with few exceptions, the stable disease patients generally have PSA maximums which do not exceed 15 pg/mL.

### Example 4G: Univariate Analysis of Number of Doublings vs. Patient Sub-Population (Recurrence or Stable Disease):

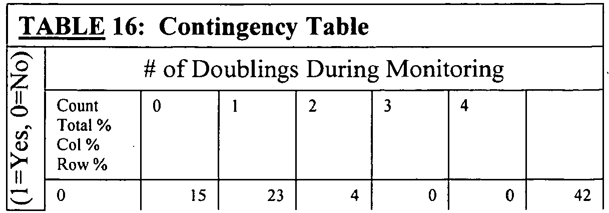

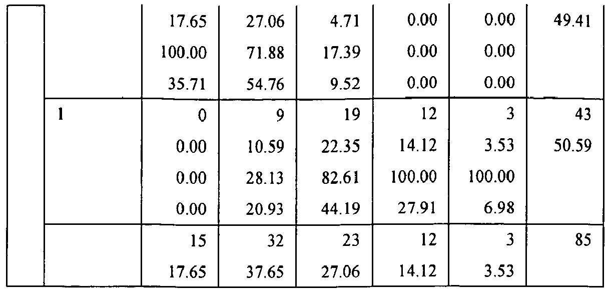

Table 16 above demonstrates that the number of doublings is increased for the 43 patients with prostate cancer recurrence versus the 42 patients with stable disease. The difference was significant at p<0.0001 (Chi-square). There is some overlap between sub-populations in the areas of 1 and 2 doublings. The degree of overlap is approximately 60% of the overall population, but it is of interest that (a) a doubling is always observed for recurrence and (b) there are no patients with 3 or 4 doublings in stable disease. A mosaic plot of the data showing the number of doublings during monitoring vs. the patient subpopulation of recurrence of prostate cancer (1) or with stable disease (0) is shown in Figure 18.

### Example 4H: Univariate Analysis of Number of Consecutive Doublings vs. Patient Sub-Population (Recurrence or Stable Disease):

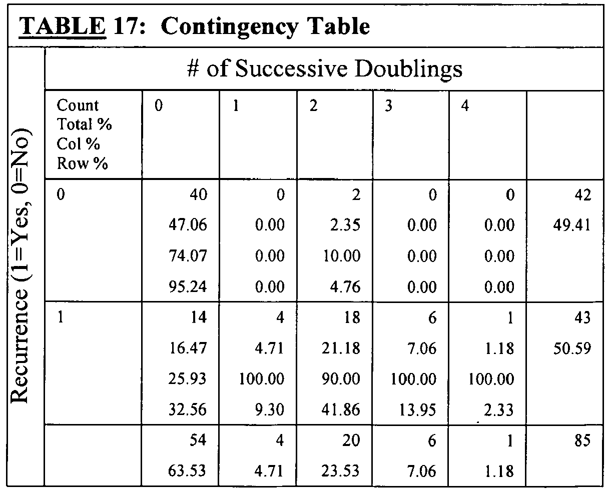

Table 17 above demonstrates that the number of consecutive doublings is increased in the 43 patients with prostate cancer recurrence vs. the 42 patients with stable disease. The difference was significant at p<0.0001 (Chi-square). The degree of overlap is approximately 80% of the overall population. A mosaic plot of the data showing the number of consecutive doublings vs. the patient subpopulation of recurrence of prostate cancer (1) or with stable disease (0) is shown in Figure 19.

### Example 5: Indicator evaluation using univariate logistic regression and receiver-operating characteristic (ROC) analysis:

Univariate logistic regression and receiver operating characteristic (ROC) curve analyses were used in evaluating whether various indicators based on PSA measurements (first post-prostatectomy PSA level, nadir PSA level, maximum observed PSA level, number of doublings, number of successive doublings, 2^{nd} pg/mL/month rise) were predictive of disease outcome. The clinical classification of patients as stable or having recurring disease was used as a reference. Additionally, for calculation of doubling time, statistical analysis showed that exponential and other fits were appropriate for 40 of 43 recurring patients and 10 of 42 stable disease patients. Exponential parameters were taken for doubling time calculations if R2 was at least ∼0.5, even if other fits gave a better fit. In addition, the tPSA values must have been rising with time for calculation of doubling time.

To assess the ability of candidate NADIA® assay indicators to predict biochemical recurrence of prostate cancer, logistic regression and ROC analyses were employed. Logistic regression models taking each candidate indicator separately (in its own model) including maximum observed value, doubling time, maximum observed PSA levl /nadir level ratio, 2^{nd} pg/mL/month, and number of doublings, were used to generate Odds Ratios (a measure of treatment effect that compares the probability of a type of outcome in the treatment group with the outcome of a control group; odds ratios deviating significantly from a value of 1.0 are desired) and p-values from the Wald test. ROC analysis provided point estimates of the area under the ROC curve (plotted as Sensitivity vs. 100-Specificity; an area of 1.0 is ideal) and the associated 95% confidence intervals (95% CIs), the best discriminating indicator value, and the associated Sensitivity and Specificity at the best discriminating indicator value. The results are summarized in Tables 18 and 19, below.

### Summary of Results of Univariate Analyses

**Table 18: Summary of Univariate Logistic Regression and ROC Results:**

| **Parameter** | **AUC** | **Wald p** |
|---|---|---|
| Maximum observed value | 0.994 | 0.0009 |
| Doubling time | 0.992 | |
| Max/Nadir | 0.973 | 0.0002 |
| pg/mL/month | 0.968 | 0.0444 |
| Number doublings | 0.902 | |

**TABLE 19:- Summary of Univariate Logistic Regression and ROC Results**

| Parameter | Odds Ratio | Wald p-value | ROC-AUC | AUC 95% Cl | Discriminating Cutpoint | Sensitivity /Specificity at cutpoint |
|---|---|---|---|---|---|---|
| Doubling time | | | 0.992 | 0.914-1.000 | 545.8 days | 93%/100% |
| Maximum observed value | 1.0657 | 0.0009 | 0.994 | 0.994-0.996 | 25.1pg/mL/mo | 98%/98% |
| Max/Nadir ratio | 1.4718 | 0.0002 | 0.973 | 0.911-0.996 | 6.1 | 95%/95% |
| 2^{nd} Rise pg/mL/month | 1.0516 | 0.0444 | 0.968 | 0.905-0.994 | 0.6 pg/mL/mo | 95%//98% |
| # Doublings | | | 0.902 | 0.818-0.956 | 1 | 79%/90% |

Areas under the ROC curves were close to the ideal state of 1.0 and the combinations of sensitivity and specificity were high except for the indicator of # of doublings. The logistic regression models for doubling time and # of doublings failed to converge due to limitations of observations. Thus, the strongest indicators of sub-populations (stable disease and early stage biochemical recurrence) were maximum observed level, the maximum PSA level/nadir level ratio, and the 2^{nd} pg/mL/month increase in NADIA® assay PSA levels. All these indicators were significant predictors of biochemical recurrence (all Wald p values were <0.05).

### Example 6: Indicator evaluation using multivariate logistic regression and ROC

To further assess the candidate indicators found to be strong predictors in univariate analysis (maximum observed level, maximum level/nadir ratio, and 2^{nd} pg/mL/month increase in NADIA® assay PSA), multivariate logistic regression and ROC analyses were performed. The intent was to determine if the NADIA® assay candidate indicators were able to maintain predictive capability even in the presence of clinicopathological prognostic indicators within the models. These clinicopathological indicators all had been previously shown to be significant predictors of recurrence and included: surgical margin involvement; capsular invasion of cancer; and peri-prostatic tissue invasion of cancer.

For each model, Odds Ratio and Wald p-value are provided for the NADIA® assay indicator and the clinicopathological indicators. The overall area under the curve (AUC) of the ROC and it's associated 95% CI are also presented. Additionally, the significance of the difference between the AUC for the multivariate model vs. the AUC for the univariate model of the NADIA® assay indicator was determined statistically. If the p-value for this statistical interpretation was <0.05 it would indicate that the multivariate model displayed increased predictive power over the NADIA® assay indicator by itself and conversely p-values >0.05 would indicate that the NADIA® assay indicator is a powerful and independent predictor and that adding clinicopathological indicators to the model does not significantly improve predictive capability for detection of prostate cancer recurrence.

The following figures and tables present the multivariate ROC curves in comparison to the univariate ROC curves employing the NADIA® assay indicator only, and the results of the logistic regression and ROC computations.

### Example 6A: Multivariate Results-Maximum Observed PSA:

**TABLE 20:**

| **Term** | **Odds Ratio** | **Wald p-value** | **ROC-AUC** | **AUC 95% Cl** | **p vs. Max by itself** |
|---|---|---|---|---|---|
| NADIA Maximum | 1.066 | 0.0497 | 0.996 | 0.918-1.000 | 0.797 |
| Surgical margins (categorical) | 236.3 | 0.0962 | | | |
| Peri-Prost Tissue invasion (categorical) | 19.5 | 0.7478 | | | |
| Capsular invasion (categorical) | 0.0042 | 0.5700 | | | |

Figures 20A and 20B show the multivariate ROC curve in comparison to the univariate ROC curve for the NADIA® maximum observed [PSA] level. Figure 20A shows the multivariate ROC curve. Figure 20B shows the univariate ROC curve for the NADIA® maximum observed [PSA] level (black line) vs. the multivariate ROC curve (dotted line). Table 20 shows the results of the logistic regression and ROC computations. A logistic regression model for maximum observed [PSA] value was used to generate Odds Ratios and p-values from the Wald test. ROC analysis provided point estimates of the area under the curve (AUC) and it's associated 95% CI are also presented.

The NADIA® maximum observed PSA level is an independent and significant predictor of outcome (p=0.0497) and the multivariate model does not significantly improve AUC (p=0.797) compared to using the parameter by itself.

### Example 6B: Multivariate Results-Maximum tPSA/Nadir Ratio:

**TABLE 21:**

| **Term** | **Regression Coeff.** | **SE** | **Odds Ratio** | **Wald p-value** | **ROC-AUC** | **AUC 95% Cl** | **p v.s. Max/Nadir by itself** |
|---|---|---|---|---|---|---|---|
| NADIA Max/Nadir ratio | 0.2764 | 0.098 | 1.3184 | 0.0051 | 0.963 | 0.866-0.995 | 0.191 |
| Surgical margins (categorical) | 1.7221 | 1.04 | 5.5964 | 0.0982 | | | |
| Peri-Prost Tissue invasion (categorical) | 1.0151 | 1.28 | 2.7597 | 0.4277 | | | |
| Capsular invasion (categorical) | 1.1495 | 2.03 | 3.1567 | 0.5711 | | | |

Figures 21A and 21B show the multivariate ROC curve in comparison to the univariate ROC curve for the NADIA® maximum total [PSA]/nadir [PSA] levels. Figure 21A shows the multivariate ROC curve. Figure 21B shows the univariate ROC curve for the NADIA® maximum total [PSA]/nadir [PSA] levels (black line) vs. the multivariate ROC curve (dotted line). Table 21 shows the results of the logistic regression and ROC computations. A logistic regression models for maximum total [PSA]/nadir [PSA] levels was used to generate Odds Ratios and p-values from the Wald test. ROC analysis provided point estimates of the area under the curve (AUC) and it's associated 95% CI are also presented.

The ratio of the maximum observed PSA level to the nadir PSA level is an independent predictor of outcome (p=0.0051) and the multivariate model does not significantly improve AUC (p=0.191) compared to using maximum observed PSA/nadir by itself.

### Example 6C: Multivariate Results-Second Rise (pg/mL/month)

**TABLE 22:**

| **Term** | **Odds Ratio** | **Wald p-value** | **ROC-AUC** | **AUC 95% Cl** | **p vs. pg/mo by itself** |
|---|---|---|---|---|---|
| NADIA pg/ml/month | 4.4250 | 0.0023 | 0.991 | 0.924-0.995 | 0.701 |
| Surgical margins (categorical) | 16.1609 | 0.0553 | | | |
| *Model did not converge when Peri-Prost Tissue Invasion and Capsular Invasion were included.* | | | | | |

Figures 22A and 22B show the multivariate ROC curve in comparison to the univariate ROC curve for the second rise in [PSA] (pg/mL/month). Figure 22B shows the multivariate ROC curve. Figure 22A shows the univariate ROC curve for the NADIA® second rise in [PSA] (pg/mL/month) (black line) vs. the multivariate ROC curve (dotted line). Table 22 shows the results of the logistic regression and ROC computations. A logistic regression models for second rise in [PSA] (pg/mL/month) was used to generate Odds Ratios and p-values from the Wald test. ROC analysis provided point estimates of the area under the curve (AUC) and it's associated 95% CI are also presented.

The second rise (pg/mL/month) is an independent predictor of outcome (p=0.0023) and the multivariate model does not significantly improve AUC (p=0.701) compared to using the second rise by itself.

### Example 7: Evaluation of [PSA] indicators as binary categorical representations

Univariate logistic regression and ROC analyses to evaluate the use of maximum observed PSA level, the maximum level/nadir level ratio, and the second rise (pg/mL/month) as binary categorical representations was also performed. Results are shown in Table 23. Indicator cutoffs for the binary categorical representations were 25 pg/mL maximum observed level, 0.6 pg/mL/month value for second rise and a maximum observed PSA level/nadir level ratio of 6. Each patient was categorized as either exceeding or not exceeding these cutoffs.

**TABLE 23--BINARY CATEGORICAL REPRESENTATIONS**

| **Univariate Logistic Regression for: Maximum observed value post-prostatectomy (Binary)** | | | | | | |
|---|---|---|---|---|---|---|
| **Term** | **Regression Coeff.** | **SE** | **Odds Ratio** | **Wald p-value** | **ROC-AUC** | **AUC 95% Cl** |
| NADIA Maximum | -6.6821 | 1.25 | 0.0013 | <0.0001 | 0.963 | 0.897-0.992 |
| | | | | | | |

| **Univariate Logistic Regression for: Max/Nadir (Binary)** | | | | | | |
|---|---|---|---|---|---|---|
| **Term** | **Regression Coeff.** | **SE** | **Odds Ratio** | **Wald** | **p-value ROC-AUC** | **AUC 95% Cl** |
| NADIA Max/Nadir ratio | -5.5053 | 0.94 | 0.0041 | <0.0001 | 0.938 | 0.862-0.979 |
| | | | | | | |

| **Univariate Logistic Regression for: pg/ml/month (Binary)** | | | | | | |
|---|---|---|---|---|---|---|
| **Term** | **Regression Coeff.** | **SE** | **Odds Ratio** | **Wald p-value** | **ROC-AUC** | **AUC 95% Cl** |
| NADIA pg/ml/month | -6.734 | 1.24 | 0.0012 | <0.0001 | 0.965 | 0.900-0.992 |

As shown in Figures 23A-C, the univariate analysis for each [PSA] indicator showed that the binary representations of these [PSA] indicators were all very powerful, with p-values < 0.0001 and AUC values approaching 1.0.

### Conclusions on the Study:

The NADIA® tPSA assay having a detection limit at least as low as 0.2 pg/mL and a functional sensitivity at least as low as 0.5 pg/mL can reliably measure tPSA concentration as low as 0.5pg/mL providing precise PSA nadir results and PSA-doubling time calculations. Measurement of tPSA using a PSA assay having a low functional sensitivity at least as low as 0.5 pg/mL, such as NADIA® assays, showed that the group of stable disease patients has a low and constant level of PSA with an approximate mean of 3.5 PG/ML (0.0035 ng/Ml). The difference between the patients having stable disease and the patients having biochemical recurrence is highly statistically significant.

In addition, on average, NADIA® assays detected a rising tPSA 34 months before the tPSA value reached 100pg/mL (0.1ng/mL).

The maximum observed PSA level is a very powerful indicator of stable disease or biochemical recurrence subpopulations. The maximum observed PSA level obtained using a PSA assay having a functional sensitivity at least as low as 0.5 pg/mL can be used to.detect biochemical relapse early. The pg/mL/month increase is also a very powerful indicator of subpopulations having stable disease or biochemical recurrence subpopulations. The ratio of maximum observed PSA level to the nadir level is also a very powerful indicator of subpopulations having stable disease or biochemical recurrence.

The NADIA® assay study showed that the tPSA parameters which served as the most discriminating indicators of sub-populations (stable disease and early stage biochemical recurrence) were the maximum observed level, 2nd consecutive pg/mL/month increase rate, and doubling time.

### Example 8: Calculations of significance of differences for patients for whom earlier data was not available

Calculation of the number of days required to reach [PSA] of 10, 25, 100, and 200 pg/mL when tPSA was measured using NADIA® assays on the sample set was performed based on exponential fitting of 40 recurring and 10 stable disease patients. This type of analysis enables a comparison of recurring and stable disease populations at very early time points following radical prostatectomy. In this retrospective study, extrapolation based on the available data fit exponentially led to greater percentage error in the determination of small values associated with the time required for recurring patients to reach 10 pg/mL PSA. However, the results for the time required to reach 25, 100, and 200 pg/ml displayed increased confidence. Wilcoxon rank sum analysis was used to determine the significance of the differences between the two sub-populations. As shown in Table 24, below, the specified levels of [PSA] were reached significantly earlier in the recurring disease population than the stable disease population.

**TABLE 24: Days required to reach various pg/ml PSA levels based on exponenetial fitting**

| **Population** | **Days to reach 10 pg/ml, Mean (SD)** | **Days to reach 25 pg/ml, Mean (SD)** | **Days to reach 100 pg/ml, Mean (SD)** | **Days to reach 200 pg/ml, Mean (SD)** |
|---|---|---|---|---|
| Total (N=50) | 491.6 (1396.2) | 1147.8 (1834.7) | 2140.7 (2600.0) | 2637.2 (3003.6) |
| Recurring (N=40) | -26.7 (762.5) | 394.4 (745.0) | 1031.5 (833.7) | 1350.0 (920.1) |
| Stable (N=10) | 2564.7 (1457.7) | 4161.6 (1818.3) | 6577.7 (2539.6) | 7785.7 (2938.3) |
| p, Recurring v. Stable* | <*0.0001* | <*0.0001* | <*0.0001* | <*0.0001* |

| | | | | |
|---|---|---|---|---|
| **Wilcoxon rank sum* | | | | |

Calculation of NADIA® assay pg/mL PSA at various time points (3, 6, 9, 12, and 18 months) were based on exponential fitting of 40 recurring and 10 stable disease patients. Wilcoxon rank sum analysis was used to determine the significance of the differences between the two sub-populations. As shown in Table 25, below, all of the values at a given point in time are higher in the recurring subpopulation than in the stable disease subpopulation. The significance of the difference increases with time, finally reaching p < 0.001 at 18 months. This indicates that the populations consistently diverge with time.

**TABLE 25: NADiA PSA pg/ml at various time points calculated by exponential fitting**

| **Population** | **pg/ml at 3 months, Mean (SD)** | **pg/ml at 6 months, Mean (SD)** | **pg/ml at 9 months, Mean (SD)** | **pg/ml at 12 months, Mean (SD** | **pg/ml at 18 months, Mean (SD)** |
|---|---|---|---|---|---|
| Total (N=50) | 40.7 (153.1) | 53.3 (208.3) | 71.0 (284.0) | 97.1 (387.6) | 228.8 (833.9) |
| Recurring (N=40) | 50.2 (170.2) | 65.9 (231.8) | 87.9 (316.0) | 120.4 (431.2) | 285.0 (926.1) |
| Stable (N=10) | 3.0 (1.8) | 3.2 (2.0) | 3.4 (2.1) | 3.6 (2.3) | 4.1 (2.8) |
| p, Recurring v. Stable* | *0.0035* | *0.0012* | *0.0006* | *0.0002* | <*0.0001* |

| | | | | | |
|---|---|---|---|---|---|
| **Wilcoxon rank sum* | | | | | |

### Example 9: Use of velocity as an indicator of EC-BCR

A retrospective study was completed comparing the linear plot of [PSA] post radical prostatectomy vs time for 16 stable patients and 13 recurring patients, over a period up to eight years. This study used the NADiA assay to measure total [PSA] as described in examples 1-6. The stable patients were defined as stable if the patient had no indication of recurrence of prostate cancer during the study period. A patient was defined as recurring if they had either a positive bone scan for prostate cancer recurrence and or death due to prostate cancer. The level of [PSA] was determined using the NADiA assay over a time period of approximately eight years. A linear curve fit was calculated for each patient. An example of the linear curve fit is shown for a stable patient (#1002) (Figure 24) and for a recurring patient (#2001) (Figure 25).

The slopes for each of the patients were determined and listed in Table 26 below:

**Table 26 Slope of the linear curve for each of the stable and recurring patients is included below:**

| | **Stable Patient #** | **Slope of Linear Curve (PSA pg/ml per Month)** | **Recurrent Patient #** | **Slope of Linear Curve (PSA pg/ml per Month)** |
|---|---|---|---|---|
| 1 | 1001 | 0.001 | 2001 | 6.723 |
| 2 | 1002 | 0.016 | 2002 | 26.604 |
| 3 | 1003 | 0.024 | 2003 | 13.035 |
| 4 | 1004 | 0.012 | 2004 | 16.290 |
| 5 | 1005 | -0.086 | 2005 | 29.044 |
| 6 | 1006 | 0.106 | 2006 | 22.712 |
| 7 | 1007 | 0.003 | 2007 | 30.255 |
| 8 | 1008 | 0.049 | 2008 | 10.004 |
| 9 | 1009 | 0.020 | 2009 | 70.460 |
| 10 | 10010 | 0.472 | 20010 | 39.419 |
| 11 | 10011 | 0.022 | 20012 | 41.681 |
| 12 | 10012 | 0.005 | 20013 | 6.576 |
| 13 | 10013 | -0.075 | 20014 | 7.523 |
| 14 | 10014 | -0.006 | | |
| 15 | 10015 | 0.0001 | | |
| 16 | 10016 | 0.041 | | |
| | | | | |
| | **Max. Value** | **0.472** | | **70.46** |
| | **Min. Value** | **-0.086** | | **6.58** |
| | **Average Value** | **0.038** | | **24.641** |

The maximum slope value for the stable patient group (pg/ml-month) is 0.472, or over13 times lower than the minimum slope value of 6.58 from the group of recurring patients. The data demonstrated that using this high sensitivity assay provides a 100% discrimination between stable and recurring patients for prostate cancer, if one assumes a patient is not having a recurrence of prostate cancer post radical prostatectomy if the slope of the [PSA] vs time is less than 1. Note there was also a significant difference between the average values for each group of patients.

### Example 10: Administration of post-prostatectomy therapy based on determination of fast, medium or slow ES-BCR

[PSA] values are obtained for post-prostatectomy patients, as described above. A PSA rate value, such as doubling time is determined, in order to discriminate between further subclasses of the recurring subpopulation of patients.

The analysis of PSA doubling time permits further sorting of patients into three groups, characterized by (1) a doubling time equal to or less than about ten months, which indicates fast or rapid recurrence; (2) a doubling time of more than about ten months up to equal to or about 24 months, which indicates medium ES-BCR, and (3) characterized by a doubling time of more than about 24 months, which indicates slow ES-BCR.

Patients displaying fast recurrence are administered post-prostatectomy therapy using external radiation therapy.

Clinical observations of Gleason scores, and wound margins are obtained for patients displaying medium or slow recurrence. Patients younger than 60 years old with Gleason scores >7 and poor margins who display medium or slow recurrence are administered post-prostatectomy therapy using external radiation therapy.

Patients older than eighty years old who display slow recurrence do not receive additional therapy.

Other patients are monitored for biochemical recurrence.

The description of specific embodiments of the invention described herein are not intended to be limiting or exclusive of other embodiments falling within the scope of the invention.
The invention further includes the subject matter of the claims of WO 2009/105264 from which this application is derived, the content of which is reproduced below as numbered paragraphs.
1. A method of detecting whether a patient has early stage biochemical recurrence (ES-BCR), comprising
   a) obtaining a sample from the patient after therapy for prostate cancer;
   b) measuring the PSA level in the sample using a PSA assay having a limit of detection less than 2.0 pg/mL;
   c) using the PSA level from one or more samples to determine a PSA value;
   wherein ES-BCR is detected if the PSA value is at least or exceeds a PSA indicator and stable disease is detected if the PSA value does not exceed the PSA indicator.
2. The method of paragraph 1 wherein the PSA indicator is [PSA].
3. The method of paragraph 1 wherein the limit of detection is at least as low as 1.0 pg/mL.
4. The method of paragraph 1 or 14 wherein the limit of detection is at least as low as 0.5 pg/mL.
5. The method of paragraph 1 or 14 wherein the limit of detection is at least as low as 0.2 pg/mL.
6. The method of any of paragraphs 1, 4, or 14 where the sample is one of a serial set of samples from the patient, and the amount of PSA in each sample is determined.
7. The method of paragraph 6 wherein the PSA indicator is a rate indicator.
8. The method of paragraph 7 wherein the rate indicator is doubling time.
9. The method of paragraph 7 wherein the rate indicator is slope of Ln [PSA] vs. time.
10. The method of paragraph 7 wherein the rate indicator is velocity of increase in [PSA].
11. The method of paragraph 6 wherein the PSA indicator is maximum observed PSA level/nadir PSA level.
12. The method of paragraph 6 wherein the PSA value is [PSA].
13. The method of paragraph 6 wherein the PSA value is a second consecutive increase (pg/mL/month).
14. A method of detecting whether a patient has early stage biochemical relapse (ES-BCR), comprising
   a) obtaining a sample from the patient after therapy for prostate cancer;
   b) measuring the PSA level in the sample using a PSA assay having a limit of detection less than 2.0 pg/mL;
   c) using the PSA level from one or more samples to determine two or more PSA values;
   wherein ES-BCR is detected if each of two or more PSA values is at least or exceeds its respective PSA indicator.
15. The method of paragraph 14 wherein the limit of detection is at least as low as 1.0 pg/mL.
16. The method of paragraph 14 wherein one PSA indicator is a rate indicator.
17. The method of paragraph 14 wherein one of the PSA indicators is the slope of Ln [PSA] vs. time.
18. The method of paragraph 16 wherein the rate indicator is doubling time.
19. The method of paragraph 16 wherein the rate indicator is velocity of increase in [PSA].
20. The method of paragraph 16 wherein the rate indicator is maximum observed PSA level/nadir PSA level.
21. The method of paragraph 14 wherein the PSA value is a second or more consecutive increase (pg/mL/month).
22. The method of any of paragraphs 2 or 12 wherein [PSA] is a PSA value and the [PSA] is at least or exceeds the lowest measured [PSA] by 2X.
23. The method of any of paragraphs 1, 14, 45, or 49-60 wherein said measuring the PSA level further comprises:
   contacting the sample with a conjugate comprising a non-nucleic acid PSA binding entity and a nucleic acid marker.
24. The method of paragraph 6 wherein said measuring the PSA level further comprises:
   contacting the sample with a conjugate comprising a non-nucleic acid PSA binding entity and a nucleic acid marker.
25. The method of any of paragraphs 8 or 18 wherein the doubling time indicator is 150 days.
26. The method of any of paragraphs 8 or 18 wherein the doubling time indicator is 400 days.
27. The method of any of paragraphs 8 or 18 wherein the doubling time indicator is 550 days.
28. The method of any of paragraphs 8 or 18 wherein the doubling time indicator is 700 days.
29. The method of any of paragraphs 8 or 18 wherein the doubling time value is 150-400 days, and Type 2 ES-BCR is detected.
30. The method of any of paragraphs 9 or 17 wherein the slope of Ln [PSA] vs. time indicator is at least 0.03.
31. The method of any of paragraphs 8 or 18 wherein the doubling time value is between about 150 days and about 400 days.
32. The method of any of paragraphs 8 or 18 wherein the doubling time value is about 10 months to 24 months.
33. The method of any of paragraphs 11 or 20 wherein the maximum observed PSA/nadir is between 3 and 11.
34. The method of any of paragraphs 11 or 20 wherein the maximum observed PSA/nadir is 6.
35. The method of any of paragraphs 2 or 12 wherein the [PSA] indicator is 10 pg/mL.
36. The method of any of paragraphs 2 or 12 wherein the [PSA] indicator is 15 pg/mL.
37. The method of any of paragraphs 2 or 12 wherein the [PSA] indicator is 20 pg/mL.
38. The method of any of paragraphs 2 or 12 wherein the [PSA] indicator is 25 pg/mL.
39. The method of any of paragraphs 2 or 12 wherein the [PSA] indicator is 50 pg/mL.
40. The method of paragraph 22 wherein the [PSA] indicator is 10 pg/mL.
41. The method of paragraph 22 wherein the [PSA] indicator is 15 pg/mL.
42. The method of paragraph 22 wherein the [PSA] indicator is 20 pg/mL.
43. The method of paragraph 22 wherein the [PSA] indicator is 25 pg/mL.
44. The method of paragraph 22 wherein the [PSA] indicator is 50 pg/mL.
45. A method of detecting whether a patient has fast, medium or slow early stage biochemical recurrence (ES-BCR), comprising
   a) obtaining a serial set of blood serum samples from the patient after therapy for prostate cancer;
   b) measuring the PSA level in each sample using a PSA assay having a limit of detection of about 0.5 pg/mL;
   c) determining the doubling time and the maximum observed PSA level;
   d) determining that the doubling time is less than a doubling time indicator, thereby detecting ES-BCR; and
   e) classifying ES-BCR as rapid, medium, or slow based on the doubling time and maximum observed PSA.
46. The method of paragraph 45, wherein the doubling time is less than 150 days.
47. The method of paragraph 45, wherein the doubling time is between 150 and 400 days.
48. The method of paragraph 45, wherein the doubling time is more than 400 days.
49. A method of detecting whether a patient has early stage biochemical relapse (ES-BCR), comprising
   a) obtaining a sample from the patient after therapy for prostate cancer. where the sample is one of a serial set of samples from the patient, and the amount of PSA in each sample is determined;
   b) measuring the PSA level in the sample using a PSA assay having a limit of detection less than 2.0 pg/mL;
   c) using the PSA level from one or more samples to determine slope of Ln [PSA] vs. time;
   wherein ES-BCR is detected if the slope of Ln [PSA] vs. time value is at least or exceeds a slope of Ln [PSA] vs. time indicator, and stable disease is detected if the slope of Ln [PSA] vs. time value does not exceed the slope of Ln [PSA] vs. time indicator.
50. The method of paragraph 49 wherein the limit of detection is less than 1.0 pg/mL.
51. The method of any of paragraphs 14 or 49 wherein the limit of detection is at least as low as 0.5 pg/mL.
52. The method of any of paragraphs 14 or 49 wherein the limit of detection is at least as low as 0.2 pg/mL.
53. A method of detecting whether a patient has early stage biochemical relapse (ES-BCR), comprising
   a) obtaining a sample from the patient after therapy for prostate cancer. where the sample is one of a serial set of samples from the patient, and the amount of PSA in each sample is determined;
   b) measuring the PSA level in the sample using a PSA assay having a limit of detection less than 2.0 pg/mL;
   c) using the PSA level from one or more samples to determine velocity of increase in [PSA]; wherein ES-BCR is detected if the velocity of increase in [PSA] value is at least or exceeds a velocity of increase in [PSA] indicator, and stable disease is detected if the velocity of increase in [PSA] value does not exceed the velocity of increase in [PSA] indicator.
54. The method of paragraph 53 wherein the limit of detection is less than 1.0 pg/mL.
55. The method of paragraph 53 wherein the limit of detection is at least as low as 0.5 pg/mL.
56. The method of paragraph 53 wherein the limit of detection is at least as low as 0.2 pg/mL.
57. A method of detecting whether a patient has early stage biochemical relapse (ES-BCR), comprising
   a) obtaining a sample from the patient after therapy for prostate cancer. where the sample is one of a serial set of samples from the patient, and the amount of PSA in each sample is determined;
   b) measuring the PSA level in the sample using a PSA assay having a limit of detection less than 2.0 pg/mL;
   wherein ES-BCR is detected if the [PSA] value is at least or exceeds a [PSA] indicator and stable disease is detected if the PSA value does not exceed the PSA indicator.
58. The method of paragraph 57 wherein the limit of detection is less than 1.0 pg/mL.
59. The method of paragraph 57 wherein the limit of detection is at least as low as 0.5 pg/mL.
60. The method of paragraph 57 wherein the limit of detection is at least as low as 0.2 pg/mL.
61. A method of detecting whether a patient has fast, medium or slow early stage biochemical recurrence (ES-BCR), comprising
   a) obtaining a serial set of blood serum samples from a patient after therapy for prostate cancer;
   b) measuring the PSA level in each sample using a PSA assay having a functional sensitivity of about 0.5 pg/mL;
   c) determining a PSA rate value;
   d) determining that the PSA rate value is equal to or less than a PSA rate indicator, thereby detecting ES-BCR; and
   e) classifying ES-BCR as rapid, medium, or slow based on the PSA rate indicator.
62. The method of any of paragraphs 1, 10, 14, 19, 45, or 49-60, 92 wherein detecting ES-BCR results in therapy selected from anti-androgen therapy, radiation therapy and chemotherapy.
63. The method of any of paragraphs 1, 10, 14, 19, 45, or 49-60, 93, 94wherein detecting stable disease results in no further therapy in the absence of later detection of ES-BCR.
64. The method of paragraph 61 wherein ES-BCR is detected and ES-BCR is classified as fast results in therapy selected from anti-androgen therapy, radiation therapy and chemotherapy.
65. The method of paragraph 61 wherein ES-BCR is detected and ES-BCR is classified as medium, further comprising (f) obtaining clinical parameters, resulting in therapy selected from anti-androgen therapy, radiation therapy and chemotherapy for patients younger than an age cutoff with Gleason scores exceeding a Gleason score cutoff.
66. The method of paragraph 61 wherein ES-BCR is detected and ES-BCR is classified as slow, further comprising (f) obtaining clinical parameters, resulting in therapy selected from anti-androgen therapy, radiation therapy and chemotherapy for patients younger than an age cutoff with Gleason scores exceeding a Gleason score cutoff.
67. The method of paragraph 61 wherein detecting-stable disease results in no further therapy in the absence of later detection of ES-BCR.
68. The method of paragraph 17, wherein a second PSA indicator is [PSA], and the [PSA] cutoff is 15 pg/ml,
69. The method of paragraph 17, wherein a second PSA indicator is [PSA], and the [PSA] cutoff is 10 pg/ml,
70. The method of paragraph 17, wherein a second PSA indicator is [PSA], and the [PSA] cutoff is 5 pg/ml,
71. The method of paragraph 14, wherein a first PSA indicator is [PSA], and the [PSA] cutoff is 5 pg/ml.
72. The method of paragraph 14, wherein a first PSA indicator is [PSA], and the [PSA] cutoff is 10 pg/ml.
73. The method of paragraph 14, wherein a first PSA indicator is [PSA], and the [PSA] cutoff is 15 pg/ml.
74. The method of any of paragraphs 68, 69 or 70 wherein a [PSA] value less than the [PSA] cutoff and the slope of Ln [PSA] vs. time is less than the slope of Ln [PSA] vs. time indicator, resulting in no further therapy in the absence of later detection of ES-BCR..
75. The method of any of paragraphs 68, 69 or 70 wherein a [PSA] value less than the [PSA] cutoff and the second PSA value is less than the second PSA indicator, resulting in no further therapy in the absence of later detection of ES-BCR.
76. The method of paragraph 71 wherein the [PSA] value is less than the [PSA] cutoff of 5 pg/ml and the second PSA value is less than the PSA indicator, resulting in no further therapy in the absence of later detection of ES-BCR.
77. The method of paragraph 72 wherein the [PSA] value is less than the [PSA] cutoff of 10 ng/ml and the second PSA value is less than the PSA indicator, resulting in no further therapy in the absence of later detection of ES-BCR.
78. The method of paragraph 73 wherein the [PSA] value is less than the [PSA] cutoff of 15 ng/ml and the second PSA value is less than the PSA indicator, resulting in no further therapy in the absence of later detection of ES-BCR.
79. The method of any of paragraphs 74 and 75 further comprising monitoring the patient for ES-BCR.
80. A kit comprising:
   a) nucleic acid-anti-PSA conjugates suitable for performing a sandwich immunoassay for PSA using PCR signal detection, wherein the assay has a detection limit at least as low as 0.2 pg/mL and a limit of detection at least as low as 0.5 pg/mL.
81. A kit comprising:
   a) nucleic acid-anti-PSA conjugates suitable for performing a sandwich immunoassay for PSA using PCR signal detection, wherein the assay has a detection limit at least as low as 0.2 pg/mL and a limit of detection at least as low as 1.0 pg/mL.
82. A kit comprising:
   a) nucleic acid-anti-PSA conjugates suitable for performing a sandwich immunoassay for PSA using PCR signal detection, wherein the assay has a detection limit at least as low as 0.2 pg/mL and a limit of detection at least as low as 2.0 pg/mL.
83. The kit of any of any of paragraphs 80, 81 or 82 wherein the nucleic-acid-anti-PSA conjugates further comprise a first nucleic acid-anti-PSA conjugate and a second nucleic-anti-PSA conjugate wherein the second-nucleic-anti-PSA conjugate is bound to a solid support.
84. The kit of any of any of paragraphs 80, 81 or 82 further comprising software for determining one or more PSA values.
85. The method of any of paragraphs 46, 47, or 48 further comprising software for determining one or more PSA values.
86. A label comprising a description of a method of detecting whether a patient has early stage biochemical relapse (ES-BCR), comprising
   a) obtaining a sample from the patient after therapy for prostate cancer;
   b) measuring the PSA level in the sample using a PSA assay having a limit of detection less than 1 pg/mL;
   c) using the PSA level from one or more samples to determine a PSA value;
   wherein ES-BCR is detected if the PSA value exceeds a PSA indicator and stable disease is detected if the PSA value does not exceed the PSA indicator.
87. A method of detecting if a patient has early stage biochemical recurrence (ES-BCR) after salvage therapy for prostate cancer, comprising
   a) obtaining a samples from the patient after salvage therapy;
   b) measuring the PSA level in the sample using a PSA assay having a limit of detection of about 0.5 pg/mL;
   c) using the PSA level from one or more samples to determine a PSA value;
   wherein ES-BCR is detected if the PSA value exceeds a PSA indicator and stable disease is detected if the PSA value does not exceed the PSA indicator.
88. The method of any of paragraphs 2 or 12 wherein the [PSA] value exceeds the lowest measured [PSA] by at least 4X.
89. The method of any of paragraphs 1, 14, 45, 49, 53, 57, 61, or 87 wherein the PSA assay is a sandwich immunoassay using two nucleic acid-anti-PSA conjugates suitable for performing a sandwich immunoassay for PSA, and further comprises using PCR signal detection.
90. The method of paragraph 89 wherein the nucleic-acid-anti-PSA conjugates further comprise a first nucleic acid-anti-PSA conjugate and a second nucleic-anti-PSA conjugate wherein the second-nucleic-anti-PSA conjugate is bound to a solid support
91. The method of paragraph 89 wherein the sandwich immunoassay for PSA is a homogeneous assay.
92. The method of any of paragraphs 10, 19, 53, 54, 55, or 56 wherein the velocity of increase in [PSA] exceeds the velocity of increase in [PSA] indicator, wherein ES-BCR is detected.
93. The method of any of paragraphs 10, 19, 53, 54, 55, or 56 wherein the velocity of increase in [PSA] does not exceed the velocity of increase in [PSA] indicator, and stable disease is detected.
94. The method of any of paragraphs 10, 19, 53, 54, 55, or 56 wherein the velocity of increase in [PSA] does not exceed the velocity of increase in [PSA] indicator, resulting in no further therapy in the absence of later detection of ES-BCR.
95. The method of any of paragraph 94 further comprising monitoring the patient for ES-BCR.
96. The method of paragraph 92 wherein the velocity of increase in [PSA] indicator is about 1.5 pg/mL/month.
97. The method of paragraph 93 wherein the velocity of increase in [PSA] indicator is about 1.5 pg/mL/month.
98. The method of paragraph 94 wherein the velocity of increase in [PSA] indicator is about 1.5 pg/mL/month.
99. The method of paragraph 95 wherein the velocity of increase in [PSA] indicator is about 1.5 pg/mL/month.
100. The method of paragraph 92 wherein the velocity of increase in [PSA] indicator is about 0.5 pg/mL/month.
101. The method of paragraph 93 wherein the velocity of increase in [PSA] indicator is about 0.5 pg/mL/month.
102. The method of paragraph 94 wherein the velocity of increase in [PSA] indicator is about 0.5 pg/mL/month.
103. The method of paragraph 95 wherein the velocity of increase in [PSA] indicator is about 0.5 pg/mL/month.
104. The method of paragraph 92 wherein the velocity of increase in [PSA] indicator is about 2.0 pg/mL/month.
105. The method of paragraph 93 wherein the velocity of increase in [PSA] indicator is about 2.0 pg/mL/month.
106. The method of paragraph 94 wherein the velocity of increase in [PSA] indicator is about 2.0 pg/mL/month.
107. The method of paragraph 95 wherein the velocity of increase in [PSA] indicator is about 2.0 pg/mL/month.
108. The method of paragraph 92 wherein the velocity of increase in [PSA] indicator is about 1.0 pg/mL/month.
109. The method of paragraph 93 wherein the velocity of increase in [PSA] indicator is about 1.0 pg/mL/month.
110. The method of paragraph 94 wherein the velocity of increase in [PSA] indicator is about 1.0 pg/mL/month.
111. The method of paragraph 95 wherein the velocity of increase in [PSA] indicator is about 1.0 pg/mL/month.
112. The kit of any of any of paragraphs 80, 81 or 82 wherein the nucleic-acid-anti-PSA conjugates further comprise a first nucleic acid-anti-PSA conjugate and a second nucleic-anti-PSA conjugate wherein the second-nucleic-anti-PSA conjugate is bound to a solid support.
113. The kit of any of any of paragraphs 80, 81 or 82 wherein the sandwich immunoassay is a homogeneous assay.

## Claims

1. A method of detecting whether a patient has stable disease, comprising
a) providing two or more samples obtained from the patient within 18 months after receiving Radical Prostatectomy (RP) for prostate cancer;
b) measuring the PSA level in the samples using an assay for PSA having a limit of detection at least as low as 1.0 pg/mL;
c) using the PSA level from the samples to determine a PSA value;
wherein stable disease is detected if the PSA value does not exceed the PSA indicator,
wherein the PSA indicator is velocity of increase in [PSA] of 6.58 µg/mL/month.

2. The method of claim 1 wherein the PSA indicator is velocity of increase in [PSA] of about 2 µg/mL/month.

3. The method of claim 1 wherein the PSA indicator is velocity of increase in [PSA] of about 1.5 µg/mL/month.

4. The method of claim 1 wherein the PSA indicator is velocity of increase in [PSA] of 1.0 pg/mL/month, or less.

5. The method of claim 1 wherein the assay is a heterogeneous sandwich format assay using polymerase chain reaction (PCR) for signal generation.

6. The method of claim 1 wherein stable disease is detected wherein the method further comprising obtaining one or more clinical parameters, resulting in therapy selected from anti-androgen therapy, radiation therapy and chemotherapy for patients younger than an age cutoff with Gleason scores exceeding a Gleason score cutoff.

7. The method of claim 6 wherein stable disease is detected wherein the clinical parameters include at least one of a Gleason score, age at diagnosis, surgical margins, T-stage, tissue invasion, capsular invasion, seminal vesicle invasion, bladder neck invasion, lymph node invasion, and tumor volume.

8. A method of detecting whether a patient has stable disease following therapy for prostate cancer, comprising
a) obtaining two or more samples from the patient within 18 months after therapy for prostate cancer;
b) measuring the PSA level in the sample using a PSA assay having a limit of detection at least as low as 1.0 pg/mL;
c) using the PSA level from two or more samples to determine a first PSA value and using the PSA level from one or more samples to determine a second PSA value;
wherein stable disease is detected if each of the first and second PSA values does not exceed its respective PSA indicator.

9. The method of claim 8 wherein the rate indicator is velocity of increase in [PSA].

10. The method of claim 9, wherein stable disease is detected if the velocity of increase in [PSA] for the patient does not exceed a velocity of increase in [PSA] indicator of 1.0 pg/mL/month and the one or more other PSA values each do not exceed its respective PSA indicator.

11. The method of claim 10, wherein the patient has a velocity of increase in [PSA] of 2.0 pg/mL/month or less and stable disease is thereby detected.

12. The method of claim 10, wherein the patient has a velocity of increase in [PSA] of 1.5 pg/mL/month or less and stable disease is thereby detected.

13. The method of claim 10 wherein the second PSA indicator is a rate indicator.

14. A method of detecting whether a patient has stable disease following RP for prostate cancer, comprising
a) obtaining two or more samples from the patient within 18 months after therapy for prostate cancer;
b) measuring the PSA level in the sample using a PSA assay having a limit of detection less than 1.0 pg/mL;
c) using the PSA level from each of the two or more samples to determine velocity of increase in [PSA];
wherein the velocity of increase in [PSA] indicator is 2.0 pg/mL/month or less.

15. The method of claim 14 wherein the velocity of increase in [PSA] indicator is 1.0 pg/mL/month or less.

16. The method of claim 14 wherein the velocity of increase in [PSA] indicator is 1.5 pg/mL/month or less.

17. The method of claim 14, wherein the velocity of increase in [PSA] does not exceed the velocity of increase in [PSA] indicator, and stable disease is detected.

18. The method claim 14, wherein the velocity of increase in [PSA] does not exceed the velocity of increase in [PSA] indicator, resulting in no further therapy.

19. The method of claim 14, wherein the patient has a velocity of increase in [PSA] equal to or less than a velocity of increase in [PSA] indicator of 2.0 pg/mL/month and stable disease is thereby detected.

20. The method of any of claims 1, 8 and 14, wherein said measuring the PSA level further comprises:
contacting the sample with a conjugate comprising a non-nucleic acid PSA binding entity and a nucleic acid marker.

21. The method of claim 6 wherein the method for measuring the PSA level is a homogeneous assay.

22. The method of any of claims 1, 8, and 14, wherein the PSA assay is a sandwich immunoassay using two nucleic acid-anti-PSA conjugates suitable for performing a sandwich immunoassay for PSA, and further comprises using PCR signal detection.

23. The method of claim 22 wherein the nucleic-acid-anti-PSA conjugates further comprise a first nucleic acid-anti-PSA conjugate and a second nucleic-anti-PSA conjugate wherein the second-nucleic-anti-PSA conjugate is bound to a solid support.

24. The method of claim 22 wherein the sandwich immunoassay for PSA is a homogeneous assay.

25. The method of claim 22 wherein the sandwich immunoassay for PSA is a heterogeneous assay.

26. The method of claim 22 wherein an anti-PSA antibody in the first nucleic acid-anti-PSA conjugate is present at a concentration of 10-30 pM.

27. A method of detecting whether a patient has stable disease following therapyRP for prostate cancer, comprising
a) obtaining one or more samples from the patient within 18 months after therapy for prostate cancer;
b) measuring the PSA level in the sample using a PSA assay having a limit of detection less than 1.0 pg/mL;
c) using the PSA level from the one or more samples to determine [PSA];
wherein stable disease is detected if the PSA value does not exceed the [PSA] cutoff.

28. The method of claim 27 wherein the PSA cut-off is 5 pg/ml or 10 pg/ml.

29. The method of claim 28 wherein the [PSA] value is less than the [PSA] cutoff, resulting in no further therapy.

30. A kit comprising:
a) nucleic acid-anti-PSA conjugates suitable for performing a sandwich immunoassay for PSA using PCR signal detection, wherein the assay has a detection limit at least as low as 0.2 pg/mL and a limit of detection at least as low as 0.5 pg/mL.
